# EUROPÄISCHE PATENTSCHRIFT

(11) **EP 3 495 800 B1**
(45) Veröffentlichungstag und Bekanntmachung des Hinweises auf die Patenterteilung: **20.09.2023**
(21) Anmeldenummer: 19155602.6
(22) Anmeldetag: 09.12.2015
(51) Int. Cl.: G01N 21/17, G01N 21/552, A61B 5/145, A61B 5/1455, G01N 33/49, G01N 21/63, A61B 5/00, A61B 5/1495

(54) **VORRICHTUNG UND VERFAHREN ZUM ANALYSIEREN EINES STOFFS**
APPARATUS AND METHOD FOR ANALYZING A MATERIAL
DISPOSITIF ET PROCÉDÉ POUR ANALYSER UNE SUBSTANCE

(43) Veröffentlichungstag der Anmeldung: 12.06.2019
(62) Teilanmeldung aus: 15828640.1
(73) Patentinhaber: DiaMonTech AG, 10245 Berlin (DE)
(72) Erfinder: Bauer, Alexander, 61440 Oberursel (DE); Hertzberg, Otto, 60318 Frankfurt am Main (DE); Lubinski, Thorsten, 10245 Berlin (DE)
(74) Vertreter: Lucke, Andreas

(56) Entgegenhaltungen:
- EP-A1- 1 048 265
- DE-A1- 3 146 700
- DE-B3-102005 048 807
- DE-B3-102014 108 424
- DE-C1- 4 446 390
- US-A1- 2013 286 397

## Beschreibung

Die vorliegende Anmeldung bezieht sich auf eine Vorrichtung und ein Verfahren zum Analysieren eines Stoffs. Die hier beschriebene Vorrichtung und das hier beschriebene Verfahren können zum Beispiel zur Analyse von tierischem oder menschlichem Gewebe, in einer Ausführungsform zur Messung von Glukose bzw. Blutzucker, genutzt werden. Bekannte Verfahren zum Analysieren eines Stoffs, insbesondere zum Messen von Blutzucker, sind beispielsweise in den folgenden Druckschriften beschrieben:
- Guo et al.: "Noninvasive glucose detection in human skin using wavelength modulated differential laser photothermal radiometry", Biomedical Optics Express, Vol, 3, 2012, No. 11,
- Uemura et al.:"Non-invasive blood glucose measurement by Fourier transform infrared spectroscopic analysis through the mucous membrane of the lip: application of a chalcogenide optical fiber system", Front Med Biol Eng. 1999; 9(2): 137-153,
- Farahi et al.: "Pump probe photothermal spectroscopy using quantum cascade lasers", J. Phys. D. Appl. Phys. 45 (2012) und
- M. Fujinami et al.: "Highly sensitive detection of molecules at the liquid/liquid interface using total internal reflection-optical beam deflection based an photothermal spectroscopy", Rev. Sci. Instrum., Vol. 74, Number 1 (2003).
- (1) von Lilienfeld-Toal, H. Weidenmüller, M. Xhelaj, A. Mäntele, W. A Novel Approach to Non- Invasive Glucose Measurement by Mid-Infrared Spectroscopy: The Combination of Quantum Cascade Lasers (QCL) and Photoacoustic Detection Vibrational Spectroscopy, 38:209-215, 2005.
- (2) Pleitez, M. von Lilienfeld-Toal, H. Mäntele W. Infrared spectroscopic analysis of human interstitial fluid in vitro and in vivo using FT-IR spectroscopy and pulsed quantum cascade lasers (QCL): Establishing a new approach to non invasive glucose measurement Spectrochimica acta. Part A, Molecular and biomolecular spectroscopy, 85:61-65, 2012
- (3) Pleitez, M. et al. In Vivo Noninvasive Monitoring of Glucose Concentration in Human Epidermis by Mid-Infrared Pulsed Photoacoustic Spectroscopy Analytical Chemsitry, 85:1013-1020, 2013.
- (4) Pleitez, M. Lieblein, T. Bauer, A. Hertzberg, O. von Lilienfeld-Toal, H. Mäntele, W. Windowless ultrasound photoacoustic cell for in vivo mid-IR spectroscopy of human epidermis: Low interference by changes of air pressure, temperature, and humidity caused by skin contact opens the possibility for a non-invasive monitoring of glucose in the interstitial fluid Review of Scientific Instruments 84, 2013
- (5) M. A. Pleitez Rafael, O. Hertzberg, A. Bauer, M. Seeger, T. Lieblein, H. von Lilienfeld-Toal, and W. Mäntele. Photothermal deflectometry enhanced by total internal reflection enables non- invasive glucose monitoring in human epidermis. The Analyst, November 2014. Es liegt die Aufgabe zugrunde, eine Vorrichtung anzugeben, mit der sich ein Stoff, insbesondere ein tierisches oder menschliches Gewebe oder ein Bestandteil oder Inhaltsstoff des Gewebes, besonders einfach und kostengünstig analysieren lässt.

Diese Aufgabe wird unter anderem durch eine Vorrichtung mit den Merkmalen gemäß Anspruch 1 gelöst. Ausgestaltungen der Vorrichtung sind in Unteransprüchen angegeben.

Eine Vorrichtung nach dem Oberbegriff des Anspruchs 1 ist aus der DE 10 2014 108 424 B3 bekannt.

In der DE 3146700A1 wird ein der photoakustischen Spektroskopie verwandtes Verfahren vorgestellt, bei dem die Wärmequellen aufgrund der Absorption eines amplitudenmodulierten Lichtstrahls jedoch nicht wie bei der photoakustischen Spektroskopie mit einem Schallaufnehmer, sondern optisch nachgewiesen werden. Dazu wird ein zweiter Lichtstrahl über die Probenoberfläche geführt und seine Beeinflussung durch temperatur- bzw. druckebhängige, periodische Brechzähländerungen gemessen. Zur Führung des Messstrahls wird eine aus der ATR-Spektroskopie an sich bekannte, sogenannte ATR-Platte benutzt, die als Deckglas auf die Probe aufgelegt ist. In dieser Platte ist das Messlicht in der Nähe des kritischen Winkels für Totalreflexion in der Grenzschicht Platte/Probe geführt. Brechzahländerungen in der Grenzschicht aufgrund der Absorption des Anregungslichtes führen zu einer nachweisbaren Modulation der Intensität des Messlichtes.

US 2013/0286397 offenbart ein Spektoskopiesystem, welches ein Array von Quantenkaskadenlasern (QCLs) enthält, welches ein Array von nicht überlagerten Laserstrahlen emittiert.

Mit dem QCL-Array ist ein Linsenarray gekoppelt, welches die Laserstrahlen kollimiert, die entlang paralleler optischer Achsen in Richtung auf eine Probe propagieren. Die Strahlen bleiben innerhalb einer Arbeitsstrecke des Arrays im Wesentlichen kollimiert, aber sie können divigieren, wenn sie über größere Strecken propagieren. Die kollimierten, parallelen Strahlen können zu der Probe oder durch die Probe hindurch gerichtet werden. Alternativ können die Strahlen auf einen Punkt gerichtet werden, der auf, in der Nähe von oder innerhalb eines Targets liegt. Das Target transmittiert, reflektiert oder streut das einfallende Licht zu einem Detektor, der die detektierte Strahlung in ein elektrisches Signal umwandelt, welches kennzeichnend für das Absorptionsspektrum oder Emissionsspektrum des Targets ist.

Die EP 1 048 265 offenbart eine Vorrichtung zum Detektieren einer Substanz in einer Probe, insbesondere zur Messung von Glukose in einem Körpergewebe. Die Vorrichtung umfasst einen Halbleiterlaser zum Emittieren von Laserlicht im mittleren Infrarotbereich bei mindestens zwei diskreten Wellenlängen, die jeweils einem unterschiedlichen Maximum oder Minimum im Absorptionsspektrum der Substanz in der Probe liegen. Mithilfe eines photoakustischen Detektors werden akustische Signale detektiert, die auf die Absorption des Laserlichts zurückzuführen sind. Eine Anzeigeeinheit wertet die akustischen Signalen für jede Wellenlänge getrennt aus und berechnet ein Detektionsresultat basierend auf allen akustischen Signalen von unterschiedlichen Wellenlängen.

Die DE 44 46 390 C1 offenbart ein Verfahren zur Messung der Konzentration eines in einer Probe enthaltenen Analyten durch Ausführen der folgenden Schritte: Beleuchtung der Probe mit einer ersten und einer zweiten Strahlungssonde, wobei die Wellenlängen der Strahlungssonden derart gewählt sind, dass der Analyt die erste Strahlungssonde vergleichsweise stark, die zweite Strahlungssonde hingegen nicht oder nur vergleichsweise schwach absorbiert; Periodische Modulation der Intensitäten der beiden Strahlungssonden; und Zeitabhängige Messung der Intensität der von der Probe transmittierten Strahlung und Erzeugung eines die Intensität repräsentierenden Messsignals. Dabei erfolgt die die Modulation der Intensitäten der Strahlungssonden gegenphasig, und die Intensitäten der Strahlungssonden werden derart aufeinander abgestimmt, dass das Messsignal zeitlich konstant ist, sofern die Konzentration des Analyten einem Sollwert entspricht. Die Amplitude und/oder die Phase einer die Modulationsfrequenz aufweisenden Komponente des Messsignals bezüglich eines Referenzsignals wird bestimmt.

Die DE 10 2005 048 807 B3 offenbart eine Vorrichtung für die quantitative und qualitative Bestimmung von IR-aktiven Inhaltsstoffen in wässrigen oder nicht-wässrigen Flüssigkeiten, umfassend a) mindestens einen ersten ATR-Körper in Form eines Lichtleiters, der eine mit der zu vermessenden Flüssigkeit in Kontakt tretende Messfläche umfasst, der für die Messstrahlung transparent oder teilweise transparent ist und der eine Brechzahl aufweist, die größer ist als die der bei der Messung an die Messfläche angrenzende Flüssigkeit, mindestens einen zweiten ATR-Körper mit mindestens einer ebenen Begrenzungsfläche, die eine mit der zu vermessenden Flüssigkeit in Kontakt tretende Messfläche umfasst, der für die Messstrahlung transparent oder teilweise transparent ist und der eine Brechzahl aufweist, die größer ist als die der bei der Messung an die Messfläche angrenzende Flüssigkeit, b) mindestens einen Probenhalter, enthaltend mindestens eine Aufnahmevorrichtung für die zu vermessende Flüssigkeit mit einer Auflagefläche für diese Flüssigkeit und einem Rand, die dimensioniert ist, um einen Kontakt der Messfläche des ersten oder zweiten ATR-Körpers mit der Oberfläche der in der Aufnahmevorrichtung vorliegenden, zu vermessenden Flüssigkeit zu gewährleisten, und c) mindestens eine Positioniervorrichtung, um die Oberfläche der Flüssigkeit und die Messfläche des ersten oder zweiten ATR-Körpers reversibel in Kontakt zu bringen.

Ein wesentlicher Vorteil der Vorrichtung ist darin zu sehen, dass sich mit dieser sehr einfach und zuverlässig ein Stoff analysieren lässt.

Unter dem Begriff Licht werden hier elektromagnetische Wellen bzw. elektromagnetische Strahlung im sichtbaren Bereich, im nahen und fernen Infrarotbereich sowie um UV-Bereich verstanden.

Bei einer beispielhaften Ausgestaltung der Vorrichtung ist vorgesehen, dass
- die Detektionseinrichtung eine Einrichtung zum Empfangen des das Reaktionssignal bildenden reflektierten Messstrahls und/oder zum direkten oder indirekten Detektieren einer Ablenkung des reflektierten Messstrahls ist.

Die Vorrichtung weist ein optisches Medium auf, das mit dem Stoff, insbesondere einem ersten Bereich der Oberfläche des Stoffs, in einer Ausführungsform der Haut eines Menschen, in direktem Kontakt steht wobei die Detektionseinrichtung zur Detektion eines Reaktionssignals eine Parameteränderung des optischen Mediums, insbesondere in einem dem ersten Bereich benachbarten Bereich, infolge des Reaktionssignals, insbesondere eine Verformung und/oder Dichteänderung des optischen Mediums, als Folge einer lokalen, zeitabhängigen Erwärmung, erfasst. Das optische Medium kann aus einem optisch oder für Infrarotstrahlung oder Ultraviolettstrahlung, allgemein für den Anregungsstrahl und den Messstrahl transparenten Material, beispielweise Glas, Kristall, Zinksulfid (ZnS), Zinkselenid (ZnSe), Germanium (Ge), Silizium (Si) und Diamant oder einem transparenten Kunststoff, in einer Ausführungsform einem Polyethylen, bestehen. Eine lokale Erwärmung als Reaktion auf einen Wärmetransport von dem zu analysierenden Stoff oder einer Substanz des Stoffes in das optische Medium führt dort zu einer Veränderung, beispielsweise einer Materialverformung oder zu thermischen Spannungen oder lokalen Veränderungen im Brechungsindex, die detektierbar sind.

Der Stoff kann dabei in einer Ausführungsform das Gewebe eines Lebewesens insbesondere eines Menschen sein, wobei die Stoffoberfläche die Haut sein kann. Es können dann Substanzen in dem Gewebe analysiert oder gemessen werden.

Es kann zudem vorgesehen sein, dass die Detektionseinrichtung einen mit dem optischen Medium verbundenes oder in dieses integriertes Piezoelement als Detektor zur Erfassung einer Spannung, Verformung und/oder Dichteänderung aufweist.

Außerdem kann vorgesehen sein, dass die Detektionseinrichtung wenigstens einen Temperatursensor als Detektor zur Erfassung des Reaktionssignals aufweist. Dieser kann direkt am optischen Medium angeordnet sein oder in seiner Umgebung, in Abhängigkeit vom Messprinzip.

Vorzugsweise weist die Vorrichtung eine Einrichtung zur Intensitätsmodulierung des Anregungslichtstrahls auf.

Die Detektionseinrichtung ist zur Detektion eines zeitabhängigen Reaktionssignals in Abhängigkeit von der Wellenlänge des Anregungslichts und/oder der Intensitäts-Modulation des Anregungslichts konfiguriert.

Auch kann es vorgesehen sein, dass die Anregungssendeeinrichtung mindestens einen elektromagnetischen Anregungsstrahl in ein Stoffvolumen einstrahlt, das unterhalb eines ersten Bereiches der Oberfläche des Stoffs liegt.

Besonders bevorzugt umfasst die Anregungssendeeinrichtung zwei oder mehr Sendeelemente, insbesondere in Form eines ein- zwei- oder mehrdimensionalen Sendeelementarrays. Dies kann somit als Flächenarray von Sendeelementen oder auch als Sendeelementleiste ausgebildet sein (in einer Ausführungsform Halbleiterlaserarrays oder QCL-Arrays, wobei QCL für Quantenkaskadenlaser steht).

Zudem kann vorgesehen sein, dass die zwei oder mehr Sendeelemente jeweils einen eigenen elektromagnetischen Anregungsstrahl erzeugen und diesen in das Volumen unterhalb des ersten Bereiches einstrahlen. Die verschiedenen Anregungsstrahlen können nacheinander oder auch wenigstens teilweise gleichzeitig ausgesendet werden. Die verschiedenen Sendeelemente können auch zeitgleich mit verschiedenen Modulationsfrequenzen betrieben werden.

Die Wellenlängen der elektromagnetischen Anregungsstrahlen der zwei oder mehr Sendeelemente unterscheiden sich vorzugsweise. Die Wellenlängen sind bevorzugt derart gewählt, dass eine in dem zu analysierenden Stoff zu detektierende Substanz Strahlung dieser Wellenlängen besonders gut absorbiert. Es können auch zusätzlich oder alternativ Wellenlängen oder Wellenlängenbereiche gewählt werden, die die zu detektierende Substanz nicht absorbiert, die aber von anderen Substanzen absorbiert werden (sogenannte tolerante Wellenlängen), um die zu analysierende Substanz von anderen Substanzen abgrenzen zu können. Die Anregungssendeeinrichtung umfasst in einer Ausführungsform zwei oder mehr Laser, insbesondere in Form eines ein- oder zweidimensionalen Laserarrays, wobei auch mehrere Reihen von Laserelementen zur Platzersparnis gestaffelt und versetzt hintereinander angeordnet sein können, in einer Ausführungsform einer Laserleiste, und/oder zwei oder mehr Leuchtdioden, insbesondere in Form eines ein- oder zweidimensionalen Diodenarrays, auch tiefengestaffelt und gegeneinander versetzt, in einer Ausführungsform eines flächigen Arrays oder einer Leiste. Die Outputstrahlen der Arrays können sowohl, nahe beisammen oder parallel, für jedes Strahlelement einzeln, als auch durch bereits integrierte Optiken, denselben Strahlengang haben.

Bezüglich des Aufbaus der Vorrichtung kann die Anregungssendeeinrichtung bereits bei der Herstellung oder zumindest vor dem Einsatz im Betrieb relativ zu dem optischen Medium justiert und fixiert werden.

Das optische Medium kann zur Befestigung und/oder Ausrichtung oder Justage von einer Anregungssendeeinrichtung und/oder Elementen einer Detektionseinrichtung wenigstens eine integrierte Erhebung und/oder Ausnehmung, wie einen Steg, eine Schulter, eine aufgesetzte Halbkugel, einen aufgesetzten Quader, einen Kegel oder eine Bohrung, Nut, Mulde oder sonstige Ausnehmung aufweisen, in oder an die die genannten Elemente (die Anregungssendeeinrichtung und /oder Elemente einer Detektionseinrichtung) angelegt, angelehnt oder an denen sie justiert oder befestigt werden können. Auch das Anarbeiten von ausgerichteten Passflächen durch Bearbeiten des optischen Mediums oder in einem Gießprozess kann vorgesehen sein.

Bezüglich der Einrichtung zur Intensitätsmodulierung kann vorgesehen sein, dass diese eine elektrische oder elektromechanische Modulationseinrichtung, die mit der Anregungssendeeinrichtung elektrisch in Verbindung steht und diese insbesondere elektrisch ansteuert, umfasst oder durch eine solche gebildet ist. Die Modulationseinrichtung kann eine Intensitätsmodulierung des Anregungsstrahls, in einer Ausführungsform eine periodische Intensitätsmodulierung, weiter beispielsweise in Form von Rechteckpulsen, einer Sägezahnfunktion oder einer Sinusfunktion oder einer anderen periodischen Funktion erzeugen.

Alternativ oder zusätzlich kann die Einrichtung zur Intensitätsmodulierung wenigstens einen im Strahlengang angeordneten gesteuerten Spiegel umfassen, durch dessen Ansteuerung die Intensität des Anregungsstrahls durch Ablenkung modulierbar ist.

Alternativ oder zusätzlich kann die Einrichtung zur Intensitätsmodulierung wenigstens eine bezüglich ihrer Transparenz steuerbare, im Strahlengang angeordnete Schicht umfassen oder durch eine solche gebildet sein. Somit kann das Modulationselement in Form eines bezüglich seiner Transmission gesteuerten Transmissionselementes ausgestaltet sein. Das Modulationselement kann aus einem Lichtstrahl mehrere, räumlich getrennte Lichtstrahlen erzeugen. Es kann in einer Ausführungsform auch vorgesehen sein, dass mit dem Modulationselement die Oberfläche einer Probe abgerastert wird. Das Modulationselement kann in einer Ausführungsform gemeinsam mit dem Array von Lichtquellen/Laserquellen gesteuert werden. Eine Einrichtung zum Aussenden eines Messstrahls, insbesondere Mess-Lichtstrahls, ist in einer Ausführungsform zum Aussenden des Messstrahls in denjenigen Bereich eines optischen Mediums vorgesehen, der mit dem ersten Bereich der Oberfläche des Stoffs in Kontakt steht.

Die Einrichtung zum Aussenden eines Messstrahls und die Detektionseinrichtung sind in einer Ausführungsform derart zueinander ausgerichtet, dass die Detektionseinrichtung als das zeitabhängige Reaktionssignal den Messstrahl detektiert, nachdem dieser zumindest einmal an derjenigen Grenzfläche des optischen Mediums reflektiert worden ist, die mit dem Stoff, insbesondere dem ersten Bereich der Oberfläche des Stoffs, in Kontakt steht.

Mit Blick auf eine einfache Montage ist es vorteilhaft, dass die Einrichtung zum Aussenden eines Messstrahls und/oder die Detektionseinrichtung und/oder die Anregungssendeeinrichtung unmittelbar mit dem optischen Medium mechanisch fest verbunden und/oder mittels eines oder mehrerer Lichtwellenleiter an dieses angekoppelt ist.

Auch sind Ausgestaltungen denkbar, bei denen das optische Medium unmittelbar eine Abbildungsoptik trägt und/oder in das optische Medium eine Abbildungs-Optik integriert ist.

Darüber hinaus sind Ausgestaltungen denkbar, bei denen die Oberfläche des optischen Mediums mehrere gegeneinander geneigte Teilflächen aufweist, an denen der Messstrahl, insbesondere der Mess-Lichtstrahl, mehrfach reflektiert wird.

Auch können Ausgestaltungen vorgesehen sein, bei denen in oder an dem optischen Medium ein oder mehrere Spiegelflächen zur Reflektion des Messstrahls, insbesondere Mess-Lichtstrahls, vorgesehen sind.

Mit Blick auf einen kompakten Aufbau ist es denkbar, dass die Anregungssendeeinrichtung und/oder die Einrichtung zum Aussenden des Messstrahls und/oder die Detektionseinrichtung unmittelbar aneinander oder an einem gemeinsamen Träger befestigt sind. Die verschiedenen Einrichtungen können an dem Träger in einer Ausführungsform durch Schwei-ßen oder Kleben oder durch Verschrauben oder durch eine Rastverbindung befestigt sein, wobei eine Justagemöglichkeit entweder beim Zusammenbau oder auch später durch eine Justierschraube oder eine andere mechanische Justiereinrichtung gegeben sein kann. Insbesondere die Einrichtung zum Aussenden des Messstrahls und/oder die Detektionseinrichtung sollten gut zueinander justiert oder justierbar sein. Darum kann es sinnvoll sein, diese beiden Einrichtungen unmittelbar an dem optischen Medium zu befestigen. Die Einrichtung zum Aussenden des Messstrahls und/oder die Detektionseinrichtung können bei geeigneter Lenkung des Messstrahls auch auf derselben Seite des optischen Mediums nebeneinander angeordnet und auf einem gemeinsamen Träger, in einer Ausführungsform einer gemeinsamen Leiterplatte oder einem gemeinsamen Halbleiter befestigt oder als gemeinsame integrierte Halbleitereinrichtung, in einer Ausführungsform als gemeinsamer integrierter Halbleiterbaustein ausgebildet sein. Dieser Träger kann dann als Ganzes gegenüber dem optischen Medium justiert werden, in einer besonderen Ausführungsform auch ohne die Relativposition zwischen der Einrichtung zum Aussenden des Messstrahls und/oder der Detektionseinrichtung noch zu verändern.

Der Träger ist bevorzugt durch eine Leiterplatte, eine Metallplatte oder Kunststoffplatte oder ein Gehäuse oder Gehäuseteil der Vorrichtung gebildet.

Es kann außerdem vorgesehen sein, dass die Anregungssendeeinrichtung einen integrierten Halbleiterbaustein umfasst, der einen oder mehrere Laserelemente sowie mindestens ein mikrooptisches Bauelement und vorzugsweise zusätzlich ein Modulationselement aufweist. Die genannten Elemente können gemeinsam aus einem Halbleiterrohling hergestellt, in einer Ausführungsform geätzt sein oder zumindest in einem gemeinsamen Gehäuse untergebracht sein.

Es kann zudem vorgesehen sein, dass das Modulationselement wenigstens ein gegenüber dem übrigen Halbleiterbaustein bewegliches und bezüglich einer Position steuerbares Element, insbesondere einen Spiegel aufweist. Dieser kann durch eine MEMS- Einrichtung ansteuerbar sein.

Es kann auch vorgesehen sein, dass das Modulationselement eine in ihrer Strahlungsdurchlässigkeit steuerbare Schicht aufweist.

Es kann auch vorgesehen sein, dass das Modulationselement eine elektronische Ansteuerschaltung für die Modulation der einen oder mehreren Laserelemente aufweist. Das Modulationselement kann in einer Ausführungsform derart beschaffen sein, dass es durch Interferenz, Phasenversatz/Gangversatz oder eine Polfiltereinrichtung oder andere bekannte Modulationsmechanismen den Anregungsstrahl zeitabhängig verändert.

Das oder die mikrooptischen Bauelemente können als in das Halbleiterbauelement integrierte oder aus diesem herausgearbeitete, insbesondere durch Ätzen herausgearbeitete Spiegel oder Linsen ausgeführt sein.

Die beschriebene Vorrichtung zum Analysieren eines Stoffes kann einen Messwert für eine Stoffkonzentration, in einer Ausführungsform eine Glucosekonzentration ermitteln. Die Vorrichtung kann eine Schnittstelle zu einer Anzeigeeinrichtung von Messwerten und ihrer Bewertung, beispielsweise durch einen Farbcode, für einen Benutzer der Vorrichtung und/oder zu einer Dosiereinrichtung für eine Substanz aufweisen, die in den Stoff, insbesondere das Gewebe oder allgemein den Körper eines Lebewesens abgegeben werden kann. Die Vorrichtung kann eine solche Dosiervorrichtung auch unmittelbar mit umfassen. Dabei kann die Vorrichtung zudem eine Einrichtung zur Erfassung oder Analyse der Stoffoberfläche, in einer Ausführungsform Hauoberfläche oder in einer Ausführungsform auch Augenfläche oder Iris eines Lebewesens aufweisen, die eine Identifizierung einer Person oder eines Lebewesens aufgrund eines Vergleiches mit Referenzdaten ermöglicht und somit dazu dienen kann, sicherzustellen, dass geeignete Referenzwerte und /oder Kalibrierungswerte für die Analyse des Stoffes und die Steuerung der Dosiervorrichtung bereitgestellt werden. Ermittelte Kennwerte der Stoffoberfläche, in einer Ausführungsform ein Fingerabdruck oder die Struktur einer Iris eines Auges, können außer zur Identifizierung und Authentifizierung einer Person, auch gegenüber einer Datenbank, ebenso zur Verschlüsselung der Kommunikation von Zustandswerten und der Steuerung der Dosiereinrichtung, die, verschlüsselt oder unverschlüsseit, grundsätzlich von der Datenbank aus geschehen kann, dienen. Die Dosiereinrichtung kann in einer Ausführungsform mit einem Sensor zur Ermittlung eines Füllstandes einer abzugebenden Substanz, wie in einer Ausführungsform Insulin und/oder Glucagon versehen sein und eine Einrichtung aufweisen, um den Füllstand zu der Vorrichtung zur Stoffanalyse und/oder direkt an die Datenbank zu übermitteln.

Zudem kann die Vorrichtung eine Schnittstelle, in einer Ausführungsform eine Funkschnittstelle zu der Datenbank aufweisen, zu der die Messwerte geschickt werden können und die die Daten weiterverarbeiten kann. Die Datenbank kann derart beschaffen sein, dass sie die Daten mehrerer Patienten , das heißt in einer Ausführungsform auch die Daten von mehreren gleichartigen Vorrichtungen zum Analysieren eines Stoffes verarbeitet und speichert und in einer Ausführungsform auch individuelle Dosiereinrichtungen zur Abgabe von Substanzen steuert. Die Datenbank kann auch die ermittelten Daten über den analysierten Stoff weiter verarbeiten und abgeleitete Analyseergebnisse wie einen Trend der werte, zeitliche erste und zweite Ableitungen, Minima, Maxima, Standardabweichungen von Stoffmengen oder - Konzentrationen, Blutzuckerwerten oder anderen physiologischen Werten von Patienten ermitteln, vergleichen und daraus Signale, in einer Ausführungsform auch Alarmsignale ableiten. Auch der Füllstand der Dosiereinrichtung kann von der Datenbank erfasst und verarbeitet werde, um in einer Ausführungsform eine zeitliche Reichweite der Füllung oder die Notwendigkeit einer Nachfüllung zu ermitteln und direkt an die Vorrichtung des Patienten oder eine Serviceeinrichtung zu signalisieren. Hierzu kann die Datenbank mit einer Kommunikationseinrichtung in einer Serviceeinrichtung, in einer Ausführungsform einem Krankenhaus oder einer Arztpraxis verbunden sein. Zum Zweck der Übermittlung von Daten von und/oder zu einer Datenbank kann die Vorrichtung in einer Ausführungsform mittels einer Funkverbindung, in einer Ausführungsform Bluetooth oder WLan oder Wifi oder anderer Übertragungsmethoden mit einem Mobilfunkgerät oder einem Pager verbunden sein. Die Vorrichtung kann auch unmittelbar mit einer WLAN- Schnittstelle und einem Internet-Client versehen sein.

Die Erfindung bezieht sich außerdem auf ein Verfahren zum Analysieren eines Stoffes, nach Anspruch 13. Dabei kann die thermische Diffusivität in dem Stoff und der zeitliche Verlauf des Reaktionssignals zur Charakterisierung der Natur des Stoffes oder einer örtlichen Verteilung einer Substanz in dem Stoff dienen oder zur Charakterisierung der Tiefe, in der der Anregungsstrahl absorbiert wird.

Es kann in einer Ausführungsform vorgesehen sein, dass nacheinander unter Verwendung verschiedener Modulationsfrequenzen der Anregungssendeeinrichtung Reaktionssignale, insbesondere zeitliche Reaktionssignalverläufe, ermittelt werden und dass mehrere Reaktionssignalverläufe zu verschiedenen Modulationsfrequenzen miteinander verknüpft werden und dass daraus insbesondere eine für einen Tiefenbereich unter der Stoffoberfläche spezifische Information gewonnen wird.

Es kann auch vorgesehen sein, dass Reaktionssignalverläufe zu verschiedenen Modulationsfrequenzen jeweils für verschiedene Wellenlängen des Anregungsstrahls ermittelt werden und daraus insbesondere jeweils eine für einen Tiefenbereich unter der Stoffoberfläche spezifische Information gewonnen wird. Bei der Verwendung von mehreren Modulationsfrequenzen des Pumpstrahls zur gleichen Zeit ist es beispielsweise möglich das detektierte Signal mittels eines entsprechenden Analyseverfahren, beispielsweise Fourier-Transformation, ihren Frequenzen entsprechend aufzulösen; die FT würde nur das Signal herausfiltern das der gewünschten Frequenz entspricht.

Es kann auch vorgesehen sein, dass ein optisches Medium mit dem Stoff, insbesondere einem ersten Bereich der Oberfläche des Stoffs, in direkten Kontakt gebracht wird, mit der Anregungssendeeinrichtung der ausgesendete Anregungsstrahl erzeugt und insbesondere derart ausgestrahlt wird, dass dieser in das optische Medium eindringt und dieses an einem vorbestimmten Punkt an der Oberfläche des optischen Mediums wieder verlässt, mit einer Einrichtung zum Aussenden eines Messstrahls ein Messstrahl, insbesondere Mess-Lichtstrahl, derart erzeugt wird, dass dieser in das optische Medium eindringt und dass insbesondere der Messstrahl und der Anregungsstrahl sich im Betrieb auf einer Grenzfläche des optischen Mediums und der Oberfläche des Stoffs, an der der Messstrahl reflektiert wird, überlappen, und mit der Detektionseinrichtung ein das Reaktionssignal bildender reflektierter Messstrahl gemessen und/oder direkt oder indirekt die Ablenkung des reflektierten Messstrahls detektiert wird.

Ein Beispiel des Verfahrens ist die Fokussierung der Messung des Reaktionssignals auf selektierte Tiefenbereiche unterhalb der (Abstandsintervalle von der) Stoffoberfläche. Die thermische Wellenlänge d hat dabei den größten Einfluss auf den mit dem Verfahren gemessenen Tiefenbereich. Sie ist definiert als d = √(D/(π^{∗}f)). Wobei D die thermische Diffusivität der Probe (hier bspw. Haut) ist und f die Modulationsfrequenz des Anregungstrahls. Literatur zur thermischen Diffusivität von Haut:
- U. Werner, K. Giese, B. Sennhenn, K. Plamann, and K. Kölmel, "Measurement of the thermal diffusivity of human epidermis by studying thermal wave propagation," Phys. Med. Biol. 37(1), 21-35 (1992).
- A. M. Stoll, Heat Transfer in Biotechnology, Vol 4 of Advances in Heat Transfer, J. P. Hartnett and T. Irvin, eds. (New York, Academic, 1967), p 117.

Zur Eliminierung von Reaktionssignalen aus den obersten Schichten des Stoffes können in einer Ausführungsform Änderungen der Messwerte im Vergleich zu vorangegangenen Messungen herangezogen werden, falls die Messwerte in den obersten Schichten sich im Vergleich zu anderen, tieferliegenden Schichten weniger oder langsamer verändern. Dies kann in einer Ausführungsform bei Messungen an der menschlichen Haut der Fall sein, wo die obersten Hautschichten praktisch keinem Austausch mit den unteren Schichten unterliegen und deshalb physiologische Parameter wenig veränderlich sind. Es kann auch die zeitliche Ableitung von Messwerten zu Reaktionssignalen zum Ausschluss der Signale aus den obersten Hautschichten herangezogen werden. So kann die Messung oder zumindest die Auswertung auf die interstitielle Flüssigkeit in der Haut begrenzt oder fokussiert werden.

Außerdem kann vorgesehen sein, dass in Abhängigkeit von einer in dem Stoff ermittelten Stoffkonzentration eine Dosiereinrichtung zur Abgabe einer Substanz, insbesondere in einen Patientenkörper angesteuert wird und/oder ein akustisches und/oder optisches Signal ausgegeben wird und/oder ein Signal mittels einer Funkverbindung an eine Verarbeitungseinrichtung abgegeben wird. Dabei können außer einem aktuell ermittelten Messwert auch eine zeitliche Messwertentwicklung, eine Ableitung des Messwertes, Durchschnittwerte von Messwerten, Maxima, Minima, eine Standardabweichung sowie vorgegebene Schwellen für Messwerte berücksichtigt und mit dem aktuellen Messwert verknüpft werden. Die Verarbeitungseinrichtung kann in einer Ausführungsform eine Datenbank sein oder mit einer Datenbank verbunden sein, die Daten mehrerer Patienten sammelt und verarbeitet. Die Datenbank kann direkt mit einer Steuerungseinrichtung der Vorrichtung verbunden oder entfernt von dieser und über eine Kommunikationsschnittstelle angebunden sein.

Um eine erhöhte Sicherheit beim Betrieb einer Dosiereinrichtung, insbesondere für Insulin, zu erreichen, kann vorgesehen sein, dass diese mittels eines voreingestellten Standardverfahrens mit vorgewählten Mengenabgaben zu bestimmten oder bestimmbaren Zeiten lokal oder von einer Datenbank aus gesteuert betrieben wird und dass mittels der oben beschriebenen Vorrichtung sinnvolle Abweichungen von voreingestellten Abgabewerten ermittelt werden, die zu einer Korrektur und Verbesserung der Ansteuerung der Dosiereinrichtung verwendet werden. Auf diese Weise ist auch bei einem Ausfall der Vorrichtung zumindest ein Normal- oder Notbetrieb der Dosiereinrichtung gewährleistet

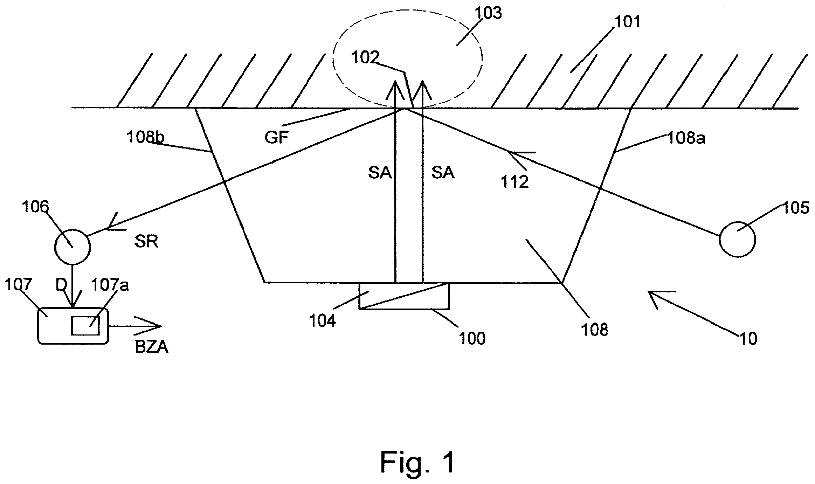

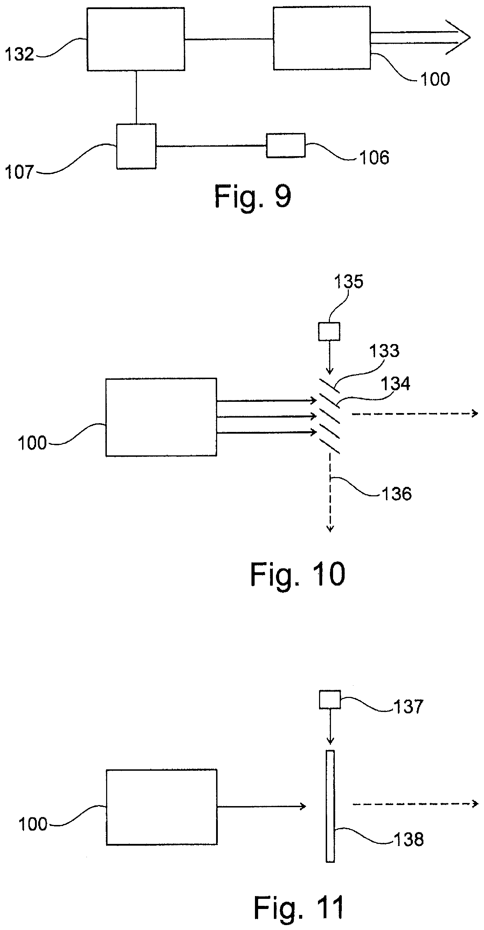

Die Figuren 1 bis 13 zeigen schematisch verschiedene Elemente der Vorrichtung und ihrer Elemente teilweise in unterschiedlichen Ausführungsformen.

Die Figur 1 zeigt ein Ausführungsbeispiel für eine Vorrichtung 10 zum Analysieren eines Stoffes 101. Der Stoff 101 liegt vorzugsweise unmittelbar auf einem optischen Medium 108 auf, das als optisch transparenter Kristall oder Glaskörper ausgebildet sein kann. Die Vorrichtung zum Analysieren des Stoffes 101 dient zum Beispiel zum Messen des Glukose- bzw. Blutzuckergehalts in einer Flüssigkeit, wie in einer Ausführungsform Blut, und zur Erzeugung einer Glukose- bzw. Blutzuckerspiegelangabe BZA.

Die Vorrichtung umfasst eine Anregungssendeeinrichtung 100 zum Aussenden eines oder mehrerer elektromagnetischer Anregungsstrahlen SA, vorzugsweise in Form von Anregungs-Lichtstrahlen mit einer oder mehreren Anregungswellenlängen, in ein Volumen 103, das in dem Stoff 101 unterhalb eines ersten Bereiches 102 der Oberfläche des Stoffes liegt. Die Anregungssendeeinrichtung 100 wird nachfolgend auch kurz als Anregungs-Lichtquelle 100 bezeichnet. Bei der Anregungs-Lichtquelle 100 kann es sich um einen bezüglich der Wellenlänge durchstimmbaren Laser, insbesondere durchstimmbaren Quantenkaskadenlaser, handeln; bevorzugt wird, wie weiter unten noch erläutert, eine Lichtquellenleiste oder ein Lichtquellenarray mit zumindest zwei Einzelemittern, insbesondere Halbleiterlasern, eingesetzt, mit denen jeweils eine vorgegebene individuelle Wellenlänge emittiert wird.

Außerdem ist eine Einrichtung 104 zur Intensitätsmodulierung des oder der Anregungslichtstrahlen SA vorgesehen, die vorzugsweise durch eine Modulationseinrichtung für die Anregungs-Lichtquelle, insbesondere ihrer Ansteuerung, und/oder wenigstens einen im Strahlengang angeordneten gesteuerten Spiegel und/oder eine bzgl. ihrer Transparenz steuerbare und im Strahlengang angeordnete Schicht gebildet ist.

Zudem weist die Vorrichtung eine Einrichtung 105 zum Aussenden eines elektromagnetischen Messstrahls 112, insbesondere Mess-Lichtstrahls, auf, der an der Grenzfläche GF zwischen dem Stoff 101 und dem optischen Medium 108 reflektiert, vorzugsweise totalreflektiert, wird.

Eine Detektionseinrichtung 106 dient zur Detektion des reflektierten Messstrahls 112, der ein zeitabhängiges Reaktionssignal SR bildet; die Amplitude des Reaktionssignals SR wird von der Wellenlänge des Anregungslichts SA und der Intensitäts-Modulation des Anregungslichts SA beeinflusst, wie weiter unten anhand von Beispielen noch näher erläutert wird.

Die Amplitude des Messsignals hängt hierbei von der Wellenlänge des Anregestrahls, den Absorptionseigenschaften der Probe, sowie den thermischen Eigenschaften, insbesondere der thermischen Diffusivität und thermischen Leitfähigkeit der Probe als auch des optischen Elementes ab. Darüber hinaus spielt auch die Kopplung des thermischen Signals von der Probe in das optische Element hinein eine Rolle.

Eine Einrichtung 107 zum Analysieren des Stoffes wertet die detektierten Reaktionssignale SR aus und erzeugt in einer Ausführungsform eine Glukose- bzw. Blutzuckerspiegelangabe BZA.

Nachfolgend soll der Betrieb der Vorrichtung 10 gemäß Figur 1 und in diesem Zusammenhang ein Verfahren zum Analysieren eines Stoffs 101 beispielhaft näher für den Fall beschrieben werden, dass es sich bei dem zu analysierenden Stoff 101 um menschliches oder tierisches Gewebe handelt und im Rahmen der Analyse des Stoffs eine Glukose- bzw. Blutzuckerspiegelangabe BZA ermittelt werden soll.

Mit der Einrichtung 105 wird ein elektromagnetischer Messstrahl 112, bei dem es sich vorzugsweise um einen Lichtstrahl im sichtbaren Wellenlängenbereich oder um einen Infrarotlichtstrahl handelt, in das optische Medium 108 eingestrahlt; dieser Messstrahl 112 trifft unterhalb des ersten Bereichs 102 der Oberfläche des Gewebes auf die Grenzfläche GF. An der Grenzfläche GF wird der Messstrahl 112 reflektiert und gelangt zur Detektionseinrichtung 106, die den reflektierten Messstrahl 112 misst.

Gleichzeitig werden mit der Anregungs-Lichtquelle 100 eine oder mehrere Anregungsstrahlen SA, bei denen es sich vorzugsweise um Infrarotstrahlen handelt, erzeugt. Die Wellenlänge des oder der Infrarotstrahlen liegt vorzugsweise in einem Bereich zwischen 3 µm und 20 µm, besonders bevorzugt in einem Bereich zwischen 8 µm und 11 µm.

Die Anregungsstrahlen SA werden mit der Einrichtung 104 zur Intensitätsmodulierung intensitäts- bzw. amplitudenmoduliert, in einer Ausführungsform werden mit der Einrichtung 104 zur Intensitätsmodulierung kurze Lichtpulse erzeugt, vorzugsweise mit einer Pulsfrequenz zwischen 1 kHz und 1 MHz oder Pulspakete (Doppel- oder Mehrfachmodulation), vorzugsweise mit einer Frequenz der Einhüllenden von 1-10 kHz.

Die modulierten Anregungsstrahlen SA werden in das optische Medium 108 eingekoppelt und gelangen nach Passieren der Grenzfläche GF in das Volumen 103 innerhalb des Gewebes.

Die Wellenlänge der Anregungsstrahlen SA ist - mit Blick auf die hier beispielhaft erläuterte Blutzuckermessung - vorzugsweise derart gewählt, dass die Anregungsstrahlen SA von Glukose bzw. Blutzucker signifikant absorbiert werden. Zur Messung von Glukose bzw. Blutzucker sind folgende Infrarotwellenlängen besonders gut geeignet (Vakuumwellenlängen): 8,1 µm, 8,3 µm, 8,5 µm, 8,8 µm, 9,2 µm, 9,4 µm und 9,7 µm. Zudem können glucosetolerante Wellenlängen verwendet werden, die von Glucose nicht absorbiert werden, um andere vorhanden Stoffe zu ermitteln und deren Einfluss auf die Messung auszuschließen.

Durch die Absorption der Anregungsstrahlen SA im Gewebe wird im Bereich des Volumens 103 eine lokale Temperaturerhöhung hervorgerufen, die einen Wärmetransport und damit einhergehend Druckwellen in Richtung auf die Grenzfläche GF auslöst; durch die dadurch an der Grenzfläche GF auftretenden Temperatur- und Druckschwankungen werden die Brechzahl bzw. die Deformation, Mikrostruktur und das Reflexionsverhalten im Bereich 102 bzw. im Reflexionsbereich der Grenzfläche GF moduliert und der Strahlengang der Messstrahlen 112 beeinflusst.

Geht man beispielsweise davon aus, dass ohne Anregungsstrahlen SA die Ausrichtung zwischen der Einrichtung 105 und der Detektionseinrichtung 106 optimal ist und eine maximale Empfangsleistung von der Detektionseinrichtung 106 detektiert wird, so kann aufgrund der Absorption der Anregungsstrahlen SA im Bereich des Volumens 103 und aufgrund des Wärmetransports und der Druckwellen eine (zumindest temporäre) Veränderung der Amplitude oder , bei einer periodischen Modulation, der Phase des reflektierten Messstrahls 112 hervorgerufen werden bzw. eine Intensitätsmodulation des reflektierten Messstrahls 112 auftreten. Der Umfang der Intensitätsmodulation hängt von der Wellenlänge der Anregungsstrahlen SA (wegen der nötigen Absorption im Gewebe) und von der Pulsfrequenz der Anregungsstrahlen SA (wegen des Temperaturtransports und der Druckwellen vom Gewebeinneren in Richtung Grenzfläche GF) und den thermischen Eigenschaften der Probe und des Mediums ab.

Die Änderung der Reflexion des Messstrahls 112 bzw. die zeitabhängige Änderung des Reaktionssignals SR wird von der Detektionseinrichtung 106 quantitativ erfasst, und das Detektionsergebnis D gelangt zur Einrichtung 107.

Die Einrichtung 107 kann anhand von vorab durchgeführten Kalibrations- bzw. Vergleichsmessungen, die in einer Ausführungsform in Form von Vergleichstabellen oder Vergleichskurven in einem Speicher 107a der Einrichtung 107 abgespeichert sind, auf die jeweils aktuelle Konzentration von Glukose bzw. Blutzucker innerhalb des Gewebes bzw. innerhalb des Volumens 103 schließen und eine entsprechende Glukose- bzw. Blutzuckerspiegelangabe BZA erzeugen. Die Vergleichstabellen oder Vergleichskurven können beispielsweise auf der Basis von Glukose- oder Blutzuckerwerten erstellt worden sein, die anhand von Blutproben ermittelt worden sind.

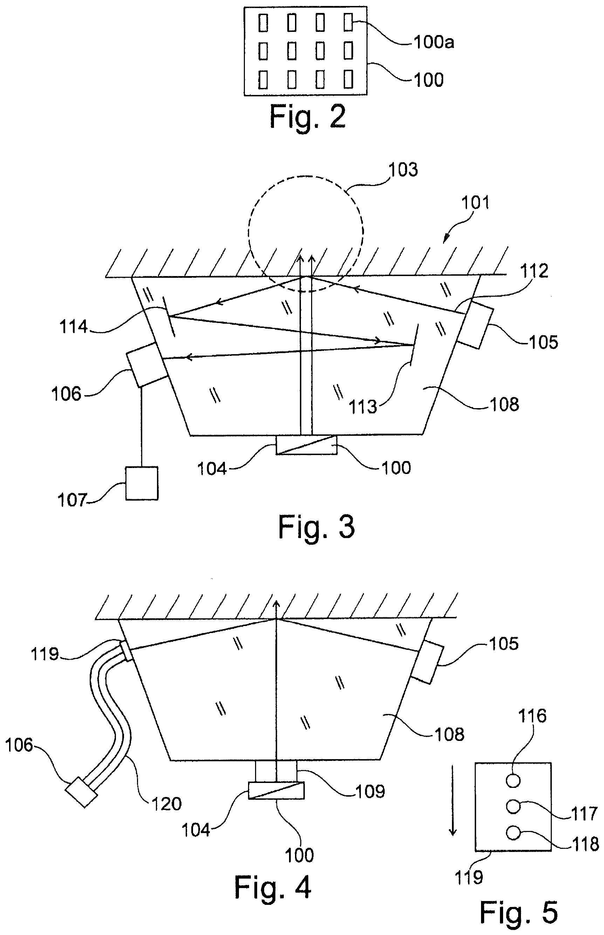

Besonders bevorzugte Ausgestaltungen und Varianten von Vorrichtungen 10 zum Analysieren eines Stoffes 101 werden nachfolgend unter Bezugnahme auf die Figuren 2 bis 10 erläutert.

Die Anregungssendeeinrichtung 100 zum Aussenden des oder der Anregungs-Lichtstrahlen kann als Array ausgebildet sein, wie in der Figur 2 dargestellt ist. Das Array weist wenigstens 5, vorteilhaft wenigstens 10, weiter vorteilhaft wenigstens 15 oder wenigstens 50 oder 100 einzeln ansteuerbare Emitter 100a für jeweils monochromatisches Licht im Absorptionsspektrum eines zu analysierenden Stoffes auf.

Das Array erzeugt vorzugsweise Strahlen mit monochromatischem Licht mit einer oder mehreren, besonders bevorzugt allen der folgenden Wellenlängen (Vakuumwellenlängen): 8,1 µm, 8,3 µm, 8,5 µm, 8,8 µm, 9,2 µm, 9,4 µm und 9,7 µm sowie, falls gewünscht zusätzlich glucosetolerante Wellenlängen.

Die Einrichtung 105 zum Aussenden des Mess-Lichtstrahls 112 und die Detektionseinrichtung 106 können von dem optischen Medium 108 separat angeordnet sein, wie in Figur 1 gezeigt. Mit Blick auf einen minimalen Platzbedarf und minimalen Montageaufwand wird es als vorteilhaft angesehen, wenn die Einrichtung 105 zum Aussenden des Mess-Lichtstrahls 112 und die Detektionseinrichtung 106 unmittelbar an dem optischen Medium 108 angebracht sind, vorzugsweise an gegenüberliegenden Flächenabschnitten 108a und 108b des optischen Mediums 108, wie dies die Figur 3 zeigt.

Es kann vorgesehen sein, dass die Anregungssendeeinrichtung/Anregungs-Lichtquelle 100 unmittelbar oder mittels einer Justagevorrichtung 109 mit dem optischen Medium 108 mechanisch fest verbunden ist. Die Justagevorrichtung 109 ermöglicht vorzugsweise eine Justage des Abstands der Anregungs-Lichtquelle 100 von dem optischen Medium 108 bzw. eine Justage in Strahllängsrichtung und/oder eine Justage in der Ebene senkrecht dazu (vgl. Figur 4).

Wie in den Figuren 3, 4, 6, 7 und 8 gezeigt, kann die Einrichtung 105 zum Aussenden des Mess-Lichtstrahls 112 in den Bereich des optischen Mediums 108 vorgesehen sein, der mit dem ersten Bereich 102 der Stoffoberfläche in Kontakt steht. Eine solche Anordnung ermöglicht das Einstrahlen des Mess-Lichtstrahls 112 in einem flachen Winkel und das Hervorrufen einer Totalreflexion an der Grenzfläche des optischen Mediums 108 zum Stoff 101.

Durch die Einstrahlung in einem flachen (kleinen) Winkel (zur Probeoberfläche) kann die Mirage Deflektion analog zu der bekannten photothermischen ,Bouncing Method' effektiver werden und gleichzeitig die deformationsbedingte Ablenkung des Messstrahls reduziert werden. Der Winkel zwischen der Probeoberfläche und dem Messstrahl kann in einer Ausführungsform kleiner als 20 Grad, kleiner als 10 Grad, insbesondere kleiner als 5 Grad, weiter insbesondere kleiner als 2 Grad oder 1 Grad gewählt werden, um diesen Effekt auszunutzen.

Umgekehrt kann durch die Einstrahlung in steileren (größeren) Winkeln (zur Stoffoberfläche) die Deflektion in Analogie zur der bekannten photothermischen ,Bouncing Method' effektiver werden und gleichzeitig die Mirage-Effekt bedingte Ablenkung des Messstrahls reduziert werden. Der Winkel zwischen der Stoffoberfläche und dem Messstrahl kann in einer Ausführungsform größer als 20 Grad, größer als 30 Grad, insbesondere größer als 45 Grad, weiter insbesondere größer als 60 Grad oder 70 Grad gewählt werden, um diesen Effekt auszunutzen.

Siehe Literatur hierzu:
- M. Bertolotti, G.L. Liakhou, R. Li Voti, S. Paolino, and C. Sibilia. Analysis of the photothermal deflection technique win the surface refection theme: Theory and Experiment. Journal of Applied Physics 83, 966 (1998)

Die Einrichtung 105 zum Aussenden des Mess-Lichtstrahls 112 und/oder die Detektionseinrichtung 106 zur Detektion des Mess-Lichtstrahls 112 bzw. des Reaktionssignals SR können tragend mit dem optischen Medium 108 mechanisch unmittelbar oder mittels einer Justageeinrichtung fest verbunden sein und/oder mittels eines oder mehrerer Lichtwellenleiter 120 an dieses angekoppelt sein.

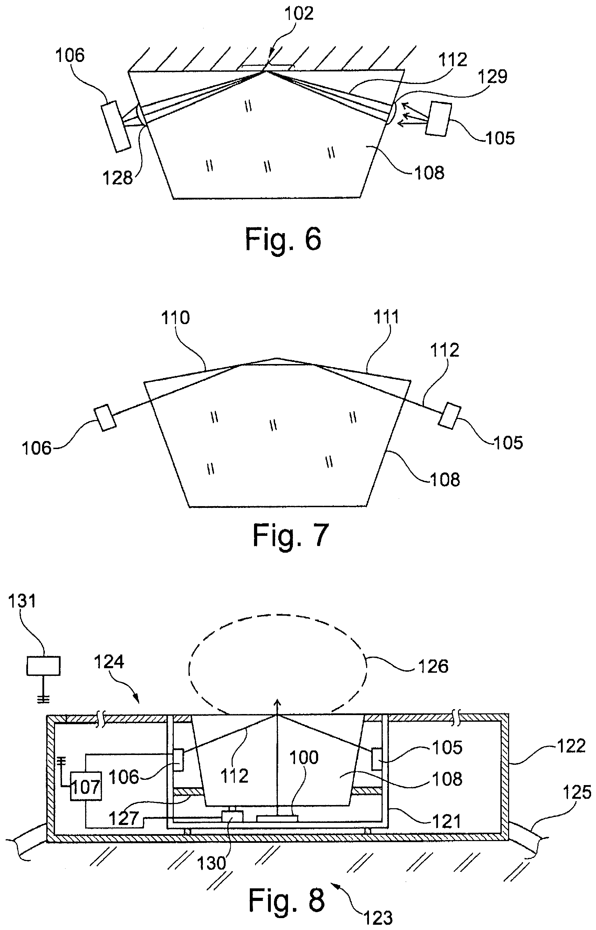

Es kann auch, wie in Figur 6 gezeigt, vorgesehen sein, dass das optische Medium 108 unmittelbar eine Abbildungsoptik 128 und/oder eine Abbildungsoptik 129 (jeweils) in Form einer Linse oder eines anderen Reflektions- oder Beugungsmittels trägt und/oder dass in das optische Medium 108 eine Abbildungs-Optik integriert ist. Die Abbildungsoptik kann jedoch auch in Form einer Linse oder eines anderen Reflexions- oder Beugungselementes in die Anregungssendeeinrichtung oder die Einrichtung zur Erzeugung des Messstrahls integriert sein, beispielsweise, wenn diese als integrierte Bauelemente und/oder als Halbleiterbauelemente ausgebildet sind. Die Abbildungsoptik kann in einer Ausführungsform aus demselben Halbleiterelement durch Ätzen herausgearbeitet sein wie der jeweilige integrierte Schaltkreis, der eine Strahlungsquelle für den Anregungs- oder Messstrahl aufweist.

Es kann auch, wie in Figur 7 gezeigt, vorgesehen sein, dass die Oberfläche des optischen Mediums 108 mehrere gegeneinander geneigte Teilflächen 110, 111 aufweist, an denen der Mess-Lichtstrahl 112 mehrfach reflektiert oder gebrochen wird.

Es kann zudem, wie in Figur 3 dargestellt, vorgesehen sein, dass in oder an dem optischen Medium 108 ein oder mehrere Spiegelflächen 113, 114 zur Reflektion des Mess-Lichtstrahls 112 (und damit des Reaktionssignals SR) vorgesehen sind. Diese Spiegelflächen können durch Inhomogenitäten innerhalb des optischen Mediums 108 oder durch dessen Außenflächen oder durch integrierte/eingepasste/eingegossene oder an dem optischen Medium befestigte, beispielsweise metallische oder metallische beschichtete, Spiegelelemente gebildet sein. Hierdurch wird der optische Weg des Mess-Lichtstrahls 112 in dem optischen Medium 108 bis zum Eintritt in die Detektionseinrichtung 106 verlängert, so dass eine reaktionssignalabhängige Ablenkung des Mess-Lichtstrahls 112 bei der Reflexion an dem Bereich der Oberfläche des Mediums 108, der mit dem ersten Bereich 102 der Stoffoberfläche im Kontakt steht, innerhalb des optischen Mediums 108 vergrößert wird. Die Ablenkung ist dann in der Detektionseinrichtung 106 als absolute Ablenkung nachweisbar.

Die Detektionseinrichtung 106 kann mehrere optisch sensitive Flächen, wie optisch sensitive Halbleiterdioden aufweisen oder auch in einem Anschlusskörper 119 mehrere versetzte Öffnungen 116, 117, 118 (Figur 5), an denen einzelne Lichtwellenleiter 120 (Figur 4) enden, in die das Licht des Messlichtstrahls 112 in Abhängigkeit von seiner Ablenkung eingekoppelt wird. Die Lichtwellenleiter 120 sind dann an einem Anschlusskörper 119, der an dem optischen Medium 108 befestigt sein kann, angeschlossen und leiten das Licht zu dem am Ende der Lichtwellenleiter 120 angeordneten Teil der Detektionseinrichtung 106 (Figur 4). Der Anschlusskörper 119 ist dann, ebenso wie die Lichtwellenleiter 120, auch ein Teil der Detektionseinrichtung 106 zur Detektion des Mess-Lichtstrahls.

Der Vollständigkeit halber soll festgestellt werden, dass auch die Anregungssendeeinrichtung die Anregung ganz oder abschnittsweise mittels eines oder mehrerer Lichtwellenleiter zu der Stoffoberfläche senden kann. in einer Ausführungsform kann die Anregungssendeeinrichtung unmittelbar mit einem oder mehreren Lichtwellenleitern gekoppelt sein die an das optische Medium angekoppelt sind.

Es kann auch, wie in Figur 8 dargestellt, vorgesehen sein, dass die Anregungssendeeinrichtung 100, die Einrichtung 105 zum Aussenden des Mess-Lichtstrahls 112 und die Detektionseinrichtung 106 unmittelbar aneinander oder an einem gemeinsamen Träger 121 befestigt sind. Der Träger kann durch ein Kunststoffteil, eine Leiterplatte oder ein Metallblech gebildet sein, das in einem Gehäuse 122 montiert ist. Der Träger, der in der Figur 8 im Querschnitt U-förmig ausgebildet ist, kann dann das optische Medium 108 in einer Ausführungsform wenigstens teilweise umgeben. Auch das optische Medium kann an dem Träger befestigt sein und gegenüber diesem justiert werden.

Der Träger kann auch durch das Gehäuse 122 selbst oder ein Gehäuseteil gebildet sein.

Es kann auch vorgesehen sein, dass die Vorrichtung mit dem Gehäuse 122 am Körper 123 einer Person befestigbar ist, wobei die Anregungssendeeinrichtung 100 zum Aussenden eines oder mehrerer Anregungs-Lichtstrahlen SA, die Einrichtung 105 zum Aussenden des Mess-Lichtstrahls 112 und die Detektionseinrichtung 106 zur Detektion des zeitabhängigen Reaktionssignals SR derart angeordnet und eingerichtet sind, dass die zur Messung geeignete Seite (mit einem für die Anregungsstrahlung transparenten Messfenster) der Vorrichtung auf der dem Körper abgewandten Seite der Vorrichtung liegt, so dass der zu analysierende Stoff auf der dem Körper 123 abgewandten Seite 124 des Gehäuses 122 vermessbar ist. Hierzu ist in der Figur 8 dargestellt, dass das Gehäuse 122 mittels eines zum Gehäuse gehörenden Riemens 125 am Körper 123 einer Person, in einer Ausführungsform in Form eines Armbandes an einem Handgelenk befestigt ist. Auf der dem Handgelenk abgewandten Seite 124 weist dann das Gehäuse ein für den Anregungs-Lichtstrahl SA durchlässiges Fenster auf, oder das optische Medium 108 ist unmittelbar in die nach außen weisende Seite 124 des Gehäuses eingepasst und bildet selbst abschnittsweise die Oberfläche des Gehäuses.

Wie in Figur 8 dargestellt, kann dann eine gestrichelt schematisiert dargestellte Fingerbeere 126 auf das optische Medium 108 aufgelegt und vermessen werden.

Das optische Medium 108 kann innerhalb des Gehäuses 122, ebenso wie der Träger 121, oder unmittelbar an dem Gehäuse 122 befestigt sein. Das optische Medium 108 kann auch unmittelbar mit dem Träger 121 verbunden sein, wobei eine Justageeinrichtung 127 zur relativen Positionierung des Trägers 121 zum optischen Medium 108 vorgesehen sein sollte.

Es ist auch denkbar, die Anregungs-Lichtquelle 100, die Einrichtung 105 und die Detektionseinrichtung 106 oder auch nur eines oder zwei der genannten Elemente unmittelbar an dem optischen Medium 108 und das oder die jeweils anderen Elemente an dem Träger 121 zu befestigen.

Durch das optische Fenster in dem Gehäuse 122 und/oder durch das optische Medium 108 hindurch können auch andere Parameter der Stoffoberfläche bzw. der aufgelegten Fingerbeere 126 vermessbar sein, wie in einer Ausführungsform ein Fingerabdruck. Hierzu kann in dem Gehäuse ein optischer Detektor 130 in Form einer Kamera beispielsweise auch an dem Träger 121 befestigt sein, die durch das optische Medium 108 hindurch ein Bild der Stoffoberfläche digital aufnimmt. Dieses Bild wird innerhalb einer Verarbeitungseinrichtung 107, die unmittelbar mit der Detektionseinrichtung und auch mit der Anregungssendeeinrichtung verbunden sein kann ebenso verarbeitet wie die Messinformationen von der Detektionseinrichtung 106. Die Verarbeitungseinrichtung kann auch Steuerungsaufgaben der Messung übernehmen. Sie kann auch wenigstens teilweise von den übrigen Teilen der Vorrichtung getrennt und entfernt sein und mittels einer Funkverbindung mit diesen kommunizieren.

Die Bilddaten von der Kamera 130 können somit innerhalb des Gehäuses oder über eine Funkverbindung auch außerhalb des Gehäuses weiterverarbeitet und mit einer Personenidentitäts-Datenbank verglichen werden, um Kalibrationsdaten der identifizierten Person abzurufen.

Derartige Kalibrationsdaten können auch entfernt (remote) in einer Datenbank, in einer Ausführungsform einer Cloud, abrufbar gespeichert sein. Auch die Messdaten der Detektionseinrichtung 106 können sowohl innerhalb als auch außerhalb des Gehäuses weiterverarbeitet werden.

Werden Daten außerhalb des Gehäuses verarbeitet, so sollten die Ergebnisdaten vorzugsweise zu der Vorrichtung innerhalb des Gehäuses zurückgefunkt werden, um dort angezeigt werden zu können.

In jedem Fall kann eine Anzeige an dem Gehäuse 122 vorgesehen sein, die vorteilhaft durch das optische Fenster, in einer Ausführungsform auch teilweise durch das optische Medium ablesbar ist. Die Anzeige kann auch durch das optische Fenster eine Leuchtanzeige auf eine Anzeigefläche projizieren und zu diesem Zweck eine Projektionsvorrichtung aufweisen. Durch die Anzeige kann in einer Ausführungsform ein Mess- oder Analyseergebnis, insbesondere eine Glucosekonzentration angezeigt werden. Die Ausgabe kann in einer Ausführungsform über einen Zeichen- oder Farbcode erfolgen. Es kann durch die Anzeige oder eine zu dieser parallele Signaleinrichtung in einer Ausführungsform ein Vorschlag für eine Insulindosis abhängig von weiteren Patientenparametern (z.B. Insulinkorrekturfaktor) oder eine automatische Signalübertragung an eine Dosiereinrichtung in Form einer Insulinpumpe erfolgen.

Die Verbindung der Vorrichtung von und zu einer externen Datenverarbeitungseinrichtung 131 kann durch alle gängigen Standards, wie Lichtleiter, Kabel, Funk (z. B. Bluetooth, WiFi), oder auch Ultraschall oder Infrarotsignale realisiert sein.

Die Figur 9 zeigt eine Modulationseinrichtung mit einer die Anregungssendeeinrichtung 100 moduliert ansteuernden Steuerung 132. Sowohl die Steuerung 132 als auch die Detektionseinrichtung 106 für den Mess-Lichtstrahl sind mit der Auswertungseinrichtung 107 verbunden.

Figur 10 zeigt eine Anregungs-Lichtquelle 100, vor der eine durch ein MEMS (Mikro-elektromechanisches System) 135 angetriebene Spiegeleinrichtung mit einem oder mehreren Mikrospiegeln 133, 134, wie sie z. B. aus der optischen Bild-Projektortechnik bekannt sind, zur zeitweiligen Umlenkung des Anregungslichtstrahls in eine Umlenkrichtung 136 angeordnet ist.

Die Figur 11 zeigt eine Anregungs-Lichtquelle 100, vor der eine optische, bezüglich der Transmission mittels einer Steuereinrichtung 137 steuerbare Schicht 138 im Anregungslichtstrahl, in einer Ausführungsform mit LCD-Zellen angeordnet ist.

Weitere Detektionsmethoden zum Nachweis eines Reaktionssignals nach Aussendung eines Anregungsstrahls können umfassen:
- Photoakustische Detektion - photoakustische Detektion mittels einer Stimmgabel oder eines anderen Schwingungselementes oder: eine leicht abgewandelte Form der Photoakustik mit offener QePAS-Zelle (Quartz enhanced PhotoAcustic Spectroskopy)). Durch diese Methoden können Druckschwankungen/schwingungen an der Stoffoberfläche nachgewiesen und ebenso ausgewertet werden, wie oben für die gemessene Strahlablenkung beschrieben.

Grundsätzlich können zur Tiefenprofilierung ermittelte Werte einer Phasenverschiebung des Reaktionssignals gegenüber einer periodischen Modulation des Anregungsstrahls genutzt werden. (Erwärmungs-/Abkühlphasen der Stoffoberfläche sollten dazu genauer bzgl. ihres Verlaufes ausgewertet werden).

Mit der beschriebenen Vorrichtung kann ein Vorrat von Klebestreifen zum Abtragen toter Hautschichten verbunden sein, um eine möglichst störungsfreie Messung an einem menschlichen Körper zu erlauben, sowie Pflaster mit Wärmeleitpaste, die auf das optische Medium regelmäßig aufgebracht werden können. Das optische Medium kann bei geeigneter Befestigung und Justierung der übrigen Teile austauschbar sein.

Die Vorrichtung kann zur Messung nicht nur an einem Finger einer Person, sondern auch an einer Lippe oder einem Ohrläppchen vorgesehen und eingerichtet sein.

Die Messung kann in einigen Ausführungen auch ohne direkte Berührung und Auflage des Fingers oder eines anderen Körperteils (auf Entfernung) funktionieren, wodurch ein kontaktloses Messen ermöglicht wird.

Die Messung kann durch Kombination mehrerer der geschilderten und erläuterten Messsysteme mit ähnlicher Fehleranfälligkeit bezüglich der Genauigkeit und Zuverlässigkeit verbessert werden.

DAQ und LockIn-Verstärker in der Auswertung können in einem Gerät zusammengefasst werden und die Auswertung kann insgesamt digitalisiert werden.

Die Messung kann mit der Vorrichtung auch an einer bewegten Stoffoberfläche durchgeführt werden, so dass im Zuge einer Rastermessung: Anregungslichtquelle und- und/oder Messlichtquelle während der Messung in einem Raster über die Haut fahren, sich ggf. Hautunregelmäßigkeiten kompensieren oder wegmitteln lassen.

Die Sensitivität der Detektionseinrichtung/Deflektionseinheit kann durch Einstellung /Variation der Wellenlänge des Probe-Beams/der Messlichtquelle optimiert werden. Hierzu kann die Messlichtquelle bzgl. der Wellenlänge veränderbar sein oder mehrere Laserlichtquellen verschiedener Wellenlänge zur Auswahl oder Kombination enthalten.

Für die Ablenkung des Pump-/Probe-Lasers kann ein optimaler transversaler Mode (TEM) gewählt werden.

Anregungssendeeinrichtung, Messlichtquelle und Detektor können als ein gemeinsamer Array aufgebaut werden und die strahlen können im optischen Medium geeignet umgelenkt werden, um Aussendung und Empfang aller Strahlen auf eine Stelle zu konzentrieren.

Eine Linse auf oder im Kristall des optischen Mediums kann dazu beitragen, den Messlichtstrahl Reaktionssignalabhängig stärker abzulenken.

Zudem ist die Verwendung einer gapfree Photodiode zur Detektion denkbar, dann könnte eine Linse den Messlichtstrahl nach Austritt bündeln und so eine genauere Messung ermöglichen.

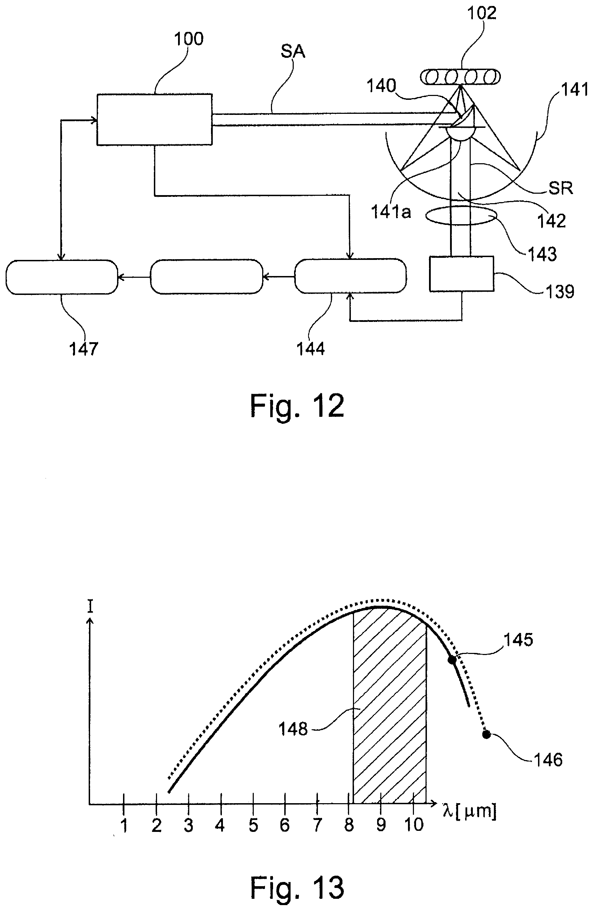

Ein Beispiel für nichtinvasive Blutzuckermessung durch eine Bestimmung der Glucose in der Haut mittels Anregung durch Quantenkaskadenlaser und Messung der Wärmewelle durch Strahlungswärme wird anhand der Figuren 12 und 13 beschrieben, mit dem die Konzentration der Glucose oder auch einem anderen Stoff in der interstitiellen Flüssigkeit (ISF) in der Haut bestimmt werden kann. Glucose in der ISF ist repräsentativ für Blutglucose und folgt ihr schnell bei Änderungen. Das Verfahren kann aus zumindest einzelnen oder Gruppen der folgenden Schritte oder aus der Gesamtabfolge bestehen:
1. Die Hautstelle 102 (in diesem Fall der erste Bereich der Stoffoberfläche) wird mit einem fokussierten und gegebenenfalls an einem Spiegel oder Hohlspiegel 140 reflektierten Strahl eines Quantenkaskadenlasers bestrahlt, der stufenweise oder kontinuierlich über einen bestimmten Infrarotbereich durchgestimmt wird, in dem Glucose spezifisch absorbiert. Anstelle des Quantenkaskadenlasers 100 kann auch ein Laserarray mit mehreren mit einzelnen Wellenlängen strahlenden Lasern verwendet werden. Der Spektralbereich kann (oder die einzelnen Wellenlängen, typisch 5 oder mehr Wellenlängen können) insbesondere zwischen ca. 900 und ca. 1300 cm⁻¹ liegen, in dem Glucose einen Absorptionsfingerprint , das heißt typische und repräsentative Absorptionslinien aufweist.
2. Der mit SA bezeichnete Anregungsstrahl wird kontinuierlich (CW-Laser) oder gepulst mit hoher Pulswiederholrate oder moduliert verwendet. Zusätzlich wird der Anregungsstrahl niederfrequent moduliert, insbesondere im Frequenzbereich zwischen 10 und 1000 Hz. Die niederfrequente Modulation kann mit verschiedenen periodischen Funktionen, in verschiedenen Ausführungsformen Sinus, Rechteck oder Sägezahn oder ähnlich erfolgen.
3. Durch die Bestrahlung der Haut nach dringt die IR-Strahlung bis etwa 50-100 µm tief in die Haut ein und regt - je nach Wellenlänge - bestimmte Schwingungen im Molekül Glucose an. Diese Anregungen vom Schwingungsniveau vo nach v1 gehen innerhalb von sehr kurzer Zeit in den Grundzustand zurück; bei diesem Schritt wird Wärme frei.
4. Als Folge der Wärmeentwicklung nach (3) bildet sich eine Wärmewelle aus, die isotrop vom Ort der Absorption ausgeht. Abhängig von der thermischen Diffusionslänge, die durch die unter (2) beschriebene niederfrequenten Modulation bestimmt wird, erreicht die Wärmewelle periodisch mit der Modulationsfrequenz die Oberfläche der Haut.
5. Das periodische Auftauchen der Wärmewelle an der Oberfläche entspricht einer periodischen Modulation der Wärmestrahlungseigenschaft der Haut (Stoffoberfläche der Probe). Die Haut kann hier näherungsweise als Schwarzer Strahler beschrieben werden, dessen Gesamtemission durch das Stefan-Boltzmann Gesetz proportional zur vierten Potenz der Oberflächentemperatur ist.
6. Mit einem Detektor 139 für Wärmestrahlung, d.h. einem Infrarotdetektor, d.h. einem Thermoelement, Bolometer, Halbleiterdetektor oder ähnlichem, der auf die Hautstelle der Einstrahlung gerichtet ist, wird die unter (5) beschriebene periodische Temperaturerhöhung registriert. Sie ist abhängig von der unter (1) und (2) beschriebenen Einstrahlung von Infrarotlicht und von der unter (3) beschriebenen Absorption und somit abhängig von der Konzentration der Glucose. Die Wärmestrahlung SR (in diesem Fall das Reaktionssignal) wird mittels eines optischen Elementes, in einer Ausführungsform einer Infrarotlinse oder eines Spiegels, insbesondere eines konkaven Parabolspiegels 141, gesammelt und, in einer Ausführungsform über einen konvexen Spiegel 141a auf den Detektor 139 geleitet. Hierzu kann ein verwendeter Sammelspiegel in einer Ausführungsform eine Öffnung 142 aufweisen, durch die der gesammelte Strahl geleitet wird. Zudem kann ein Filter 143 im Strahlengang vorgesehen sein, das nur Infrarotstrahlung eines bestimmten Wellenlängenbereichs durchlässt.
7. Es kann bei der Bearbeitung der Reaktionssignale speziell die Modulationsfrequenz berücksichtigt werden, wozu das Reaktionssignal in einem Lock- in- Verstärker 144 verarbeitet werden kann. Durch Analyse der Phasenlage zwischen Anregungssignal und Wärmestrahlungssignal (Reaktionssignal) mittels einer Steuerungs- und Verarbeitungseinrichtung 147 kann die Tiefeninformation über die Tiefe unter der Stoffoberfläche gewonnen werden, aus der die Reaktionssignale vorwiegend erhalten werden.
8. Durch die Auswahl und Analyse verschiedener niederfrequenter Modulationsfrequenzen wie unter (2) beschrieben für den Anregungsstrahl und die Verknüpfung der Ergebnisse für verschiedene Modulationsfrequenzen (wobei die Ergebnisse für verschiedene Modulationsfrequenzen auch unterschiedlich gewichtet werden können) kann ebenfalls die Tiefeninformation erhalten werden. Dabei können ggf. Differenzverfahren oder andere Bestimmungsverfahren verwendet werden, um die Absorption der obersten Hautschichten zu kompensieren.
9. Um die Detektion der Wärmestrahlung nach (6) möglichst empfindlich zu machen, wird sie spektral breitbandig für den gesamten in Frage kommenden Infrarotbereich genutzt. Dabei sollen möglichst viele Bereiche der Planckschen Strahlungskurve genutzt werden. Um die Detektion für die intensive Anregungsstrahlung unempfindlich zu machen, wird die Detektion der Wärmestrahlung mit Sperrfilter (Notch-Filter) 143 für diese Anregungswellenlängen versehen. Der durch das Sperrfilter 143 durchgelassene Wellenlängenbereich 148 ist auch aus dem Diagramm der Figur 13 ersichtlich. Dort ist zudem die Intensität des Reaktionssignals wellenlängenabhängig einmal in einer ersten (durchgezogenen) Kurve 145 ohne einen Anregungsstrahl oder nur mit Anregungsstrahlung in für den nachzuweisenden Stoff unspezifischen Wellenlängen (d.h. ohne die Wellenlängen, bei denen spezifische Absorptionsbanden des Stoffes vorliegen) dargestellt und zudem in einer zweiten (gestrichelten) Kurve 146 eine vergleichbare Kurve, wobei ein Anregungsstrahl eingestrahlt wird, der spezifische Absorptionswellenlängen des nachzuweisenden Stoffes enthält.
10. Aus dem nach (6-9) gemessenen, von der Anregungswellenlänge abhängigen Wärmesignal wird somit in einer Ausführungsform, wenn Glucose nachgewiesen werden soll, zunächst bei nicht (oder unter Ausnahme von) glucoserelevanten Wellenlängen des Anregungsstrahls (Kurve 145) der Hintergrund bestimmt, anschließend bei (oder unter Einschluss von) glucoserelevanten Wellenlängen die Differenz zum Hintergrundsignal. Daraus ergibt sich die Glucosekonzentration in der Hautschicht oder den Hautschichten, die durch die ausgewählte Phasenlage nach (7) oder die verschiedenen Modulationsfrequenzen nach (8) oder deren Verknüpfung festgelegt werden.

Obwohl die Erfindung im Detail durch bevorzugte Ausführungsbeispiele näher illustriert und beschrieben wurde, so ist die Erfindung nicht durch die offenbarten Beispiele eingeschränkt und andere Variationen können vom Fachmann hieraus abgeleitet werden, im Rahmen der angehängten Ansprüche.

### Bezugszeichenliste

- 10: Vorrichtung
- 100: Anregungssendeeinrichtung / Anregungs-Lichtquelle
- 100a: Emitter / Sendeelemente
- 101: Stoff
- 102: erster Bereich
- 103: Volumen
- 104: Einrichtung
- 105: Einrichtung
- 106: Detektionseinrichtung
- 107: Verarbeitungseinrichtung /Auswertungseinrichtung
- 107a: Speicher
- 108: optisches Medium
- 108a: Flächenabschnitt
- 108b: Flächenabschnitt
- 109: Justagevorrichtung
- 110: Teilfläche
- 111: Teilfläche
- 112: Messstrahl / Mess-Lichtstrahl
- 113: Spiegelfläche
- 114: Spiegelfläche
- 116: Öffnung
- 117: Öffnung
- 118: Öffnung
- 119: Anschlusskörper
- 120: Lichtwellenleiter
- 121: Träger
- 122: Gehäuse
- 123: Körper
- 124: Seite
- 125: Riemen
- 126: Fingerbeere
- 127: Justageeinrichtung
- 128: Abbildungsoptik
- 129: Abbildungsoptik
- 130: optischer Detektor / Kamera
- 131: Datenverarbeitungseinrichtung
- 132: Steuerung
- 133: Mikrospiegel
- 134: Mikrospiegel
- 135: Mikro-elektromechanisches System
- 136: Umlenkeinrichtung
- 137: Steuereinrichtung
- 138: Schicht
- 139: Infrarot- Detektor
- 140: Spiegel
- 141: Hohlspiegel
- 142: Öffnung in 141
- 143: Wellenlängenfilter
- 144: Lock-in -Verstärker
- 145: Signalkurve des Reaktionssignals (durchgezogen)
- 146: Signalkurve des Reaktionssignals (gestrichelt)
- 147: Steuerungs- und Verarbeitungseinrichtung
- 148: Wellenlängenbereich

- BZA: Blutzuckerspiegelangabe
- D: Detektionsergebnis
- GF: Grenzfläche
- SA: Anregungsstrahl
- SR: Reaktionssignal

## Patentansprüche

1. Vorrichtung (10) zum Analysieren eines Stoffes (101) mit
einer Anregungssendeeinrichtung (100) in Form einer Laserlichtquelle zum Erzeugen eines oder mehrerer elektromagnetischer Anregungsstrahlen (SA), mit zumindest einer Anregungswellenlänge, wobei die Anregungssendeeinrichtung (100) den einen oder die mehreren elektromagnetischen Anregungsstrahlen (SA) in ein Volumen (103) einstrahlt, das in dem Stoff unterhalb eines ersten Bereiches (102) der Oberfläche des Stoffes liegt,
einem optischen Medium (108), das mit dem ersten Bereich (102) der Oberfläche des Stoffs (101) in direktem Kontakt steht,
einer Detektionseinrichtung (106, 139) zur Detektion eines Reaktionssignals (SR), wobei die Vorrichtung (10) eine Einrichtung (105) zum Aussenden eines elektromagnetischen Messstrahls (112) aufweist, die so angeordnet ist, dass der ausgesendete Messstrahl (112) in das optische Medium (108) eindringt, und an der Grenzfläche (GF) zwischen dem Stoff (101) und dem optischen Medium (108) totalreflektiert wird, wobei die Detektionseinrichtung (106) und die Einrichtung (105) zum Aussenden des elektromagnetischen Messstrahls (112) derart zueinander ausgerichtet sind, dass die Detektionseinrichtung (106) zur Detektion des reflektierten Messstrahls, der ein zeitabhängiges Reaktionssignal (RS) bildet, wobei die Amplitude des Reaktionssignals (RS) von der Wellenlänge des Anregungsstrahls und dessen Intensitätsmodulation beeinflusst wird, konfiguriert ist, und
mit einer Einrichtung (107, 147) zum Analysieren des Stoffes anhand des detektierten Reaktionssignals (SR), **dadurch gekennzeichnet, dass**
die Anregungssendeeinrichtung (100) unmittelbar - oder mittelbar mittels einer Justagevorrichtung (109) - mit dem optischen Medium (108) mechanisch fest verbunden ist oder
die Einrichtung zum Aussenden eines Messstrahls (112) und/oder die Einrichtung (106) zum Detektieren des reflektierten Messstrahls und/oder die Anregungssendeeinrichtung (100) unmittelbar mit dem optischen Medium (108) mechanisch fest verbunden und/oder mittels eines Lichtwellenleiters (120) an dieses angekoppelt ist.

2. Vorrichtung (10) nach Anspruch 1,
**dadurch gekennzeichnet, dass**
im Betrieb der Messstrahl (112) und der Anregungsstrahl (SA) sich auf der Grenzfläche (GF) des optischen Mediums (108) und der Oberfläche des Stoffs (101), an der der Messstrahl (112) reflektiert wird, überlappen, und
die Detektionseinrichtung (106) eine Einrichtung zum direkten oder indirekten Detektieren einer Ablenkung des reflektierten Messstrahls (112) ist.

3. Vorrichtung (10) nach Anspruch 1 oder 2,
**dadurch gekennzeichnet, dass**
die Detektionseinrichtung (106) zur Detektion eines Reaktionssignals (SR) eine Verformung und/oder Dichteänderung des optischen Mediums in einem dem genannten ersten Bereich benachbarten Bereich infolge des Reaktionssignals erfasst, oder
die Detektionseinrichtung ein mit dem optischen Medium verbundenes oder in dieses integriertes Piezoelement als Detektor zur Erfassung einer Verformung oder Dichteänderung desselben aufweist, und/oder
die Detektionseinrichtung Temperatursensoren als Detektor zur Erfassung des Reaktionssignals aufweist, und/oder
die Vorrichtung eine Einrichtung (104) zur Intensitätsmodulierung des Anregungslichtstrahls (SA) aufweist und
die Detektionseinrichtung (106) zur Detektion eines zeitabhängigen Reaktionssignals (SR) in Abhängigkeit von der Wellenlänge des Anregungslichts und/oder der Intensitäts-Modulation des Anregungslichts geeignet ist.

4. Vorrichtung (10) nach einem der voranstehenden Ansprüche,
**dadurch gekennzeichnet, dass**
die Anregungssendeeinrichtung (100) zwei oder mehr Sendeelemente (100a), insbesondere in Form eines ein-, zwei- oder mehrdimensionalen Sendeelementarrays, umfasst, und
die zwei oder mehr Sendeelemente (100a) jeweils einen eigenen elektromagnetischen Anregungsstrahl erzeugen und diesen in das Volumen unterhalb des genannten ersten Bereiches (102) einstrahlen.

5. Vorrichtung (10) nach Anspruch 4,
**dadurch gekennzeichnet, dass**
sich die Wellenlängen der elektromagnetischen Anregungsstrahlen der zwei oder mehr Sendeelemente (100a) unterscheiden, oder
die Anregungssendeeinrichtung zwei oder mehr Laser, insbesondere in Form eines ein-, zwei- oder mehrdimensionalen Laserarrays, und/oder zwei oder mehr Leuchtdioden, insbesondere in Form eines ein-, zwei- oder mehrdimensionalen Diodenarrays, umfasst.

6. Vorrichtung (10) nach Anspruch 3
**dadurch gekennzeichnet, dass**
die Einrichtung (104) zur Intensitätsmodulierung eine elektrische Modulationseinrichtung, die mit der Anregungssendeeinrichtung (100) elektrisch in Verbindung steht und diese elektrisch ansteuert, umfasst oder durch eine solche gebildet ist, oder
die Einrichtung (104) zur Intensitätsmodulierung wenigstens einen im Strahlengang angeordneten gesteuerten Spiegel (133, 134) umfasst, oder
die Einrichtung (104) zur Intensitätsmodulierung wenigstens eine bezüglich ihrer Transparenz steuerbare, im Strahlengang angeordnete Schicht (138) umfasst oder durch eine solche gebildet ist.

7. Vorrichtung nach einem der vorhergehenden Ansprüche,
**dadurch gekennzeichnet, dass**
das optische Medium unmittelbar eine Abbildungsoptik (128, 129) trägt und/oder in das optische Medium eine Abbildungsoptik (128, 129) integriert ist.

8. Vorrichtung (10) nach einem der vorhergehenden Ansprüche, bei der die Oberfläche des optischen Mediums mehrere gegeneinander geneigte Teilflächen (110, 111) aufweist, an denen der Messstrahl (112), insbesondere der Mess-Lichtstrahl, mehrfach reflektiert wird.

9. Vorrichtung (10) nach einem der voranstehenden Ansprüche,
**dadurch gekennzeichnet, dass**
in oder an dem optischen Medium (108) ein oder mehrere Spiegelflächen (113, 114) zur Reflektion des Messstrahls (112), insbesondere Mess-Lichtstrahls, vorgesehen sind, oder
die Anregungssendeeinrichtung (100) und/oder die Einrichtung (105) zum Aussenden des Messstrahls und/oder die Detektionseinrichtung (106) unmittelbar aneinander oder an einem gemeinsamen Träger (121) befestigt sind.

10. Vorrichtung (10) nach Anspruch 9,
**dadurch gekennzeichnet, dass**
der Träger (121) durch eine Leiterplatte, eine Metallplatte oder Kunststoffplatte oder ein Gehäuse (122) oder Gehäuseteil der Vorrichtung gebildet ist, oder
die Anregungssendeeinrichtung einen integrierten Halbleiterbaustein umfasst, der einen oder mehrere Laserelemente sowie mindestens ein mikrooptisches Bauelement und vorzugsweise zusätzlich ein Modulationselement aufweist.

11. Vorrichtung (10) nach Anspruch 6,
**dadurch gekennzeichnet, dass**
das Modulationselement wenigstens ein gegenüber dem übrigen Halbleiterbaustein bewegliches und bezüglich einer Position steuerbares Element, insbesondere einen Spiegel aufweist, oder
das Modulationselement eine in ihrer Strahlungsdurchlässigkeit steuerbare Schicht aufweist.

12. Vorrichtung (10) nach Anspruch 6,
**dadurch gekennzeichnet, dass**
die Modulationseinrichtung eine elektronische Ansteuerschaltung für die Modulation der einen oder mehreren Laserelemente aufweist.

13. Verfahren zum Analysieren eines Stoffes (101) unter Verwendung einer Vorrichtung nach einem der Ansprüche 1 bis 12, wobei bei dem Verfahren
mit der Anregungssendeeinrichtung (100) ein oder mehrere elektromagnetische Anregungsstrahlen (SA) mit zumindest einer Anregungswellenlänge durch den wenigstens teilweise gleichzeitigen oder aufeinander folgenden Betrieb eines oder mehrerer Laseremitter einer Laserlichtquelle erzeugt und in ein Volumen (103), das in dem Stoff unterhalb eines ersten Bereiches (102) der Oberfläche des Stoffes liegt, eingestrahlt wird bzw. werden,
und wobei mit der Einrichtung (105) zum Aussenden eines elektromagnetischen Messstrahls (112) ein elektromagnetischer Messstrahl (112) ausgesandt wird, der an der Grenzfläche (GF) zwischen dem Stoff (101) und einem optischen Medium (108) totalreflektiert wird,
mit der Detektionseinrichtung (106) ein zeitabhängiges Reaktionssignal (SR) detektiert wird, wobei die Amplitude des Reaktionssignals von der Wellenlänge des Anregungsstrahls und dessen Intensitätsmodulation beeinflusst wird, und
mit der Einrichtung (107, 147) zum Analysieren des Stoffes anhand des detektierten Reaktionssignals (SR) der Stoff analysiert wird.

14. Verfahren nach Anspruch 13, **dadurch gekennzeichnet, dass** nacheinander unter Verwendung verschiedener Modulationsfrequenzen der Anregungssendeeinrichtung (100) Reaktionssignale in Form von zeitlichen Reaktionssignalverläufen ermittelt werden und dass mehrere Reaktionssignalverläufe zu verschiedenen Modulationsfrequenzen miteinander verknüpft werden und dass daraus eine für einen Tiefenbereich unter der Stoffoberfläche spezifische Information gewonnen wird,
wobei Reaktionssignalverläufe zu verschiedenen Modulationsfrequenzen jeweils für verschiedene Wellenlängen des Anregungsstrahls ermittelt werden und daraus insbesondere jeweils eine für einen Tiefenbereich unter der Stoffoberfläche spezifische Information gewonnen wird,
wobei vorzugsweise bei Verwendung von mehreren Modulationsfrequenzen des Pumpstrahls zur gleichen Zeit das detektierte Signal mittels eines Analyseverfahrens, vorzugsweise einer Fourier-Transformation, seinen Frequenzen entsprechend getrennt wird und
jeweils nur das Teilsignal herausgefiltert wird, das der gewünschten Frequenz entspricht.

15. Verfahren nach einem der voranstehenden Ansprüche 13-14,
**dadurch gekennzeichnet, dass**
mit der Detektionseinrichtung (106) ein das Reaktionssignal (SR) bildender reflektierter Messstrahl (112) gemessen wird,
oder
bei dem der Anregungsstrahl periodisch mit einer ersten Frequenz amplitudenmoduliert wird und eine Frequenzverschiebung des Reaktionssignals relativ zu der ersten Frequenz ermittelt wird oder
in Abhängigkeit von einer in dem Stoff ermittelten Stoffkonzentration eine Dosiereinrichtung zur Abgabe einer Substanz in einen Patientenkörper angesteuert wird.

## Claims

1. An apparatus (10) for analyzing a substance (101), comprising
an excitation transmission device (100) in the form of a laser light source for generating one or more electromagnetic excitation beams (SA) having at least one excitation wavelength, wherein the excitation transmission device (100) radiates the one or more electromagnetic excitation beams (SA) into a volume (103) which is located in the substance below a first region (102) of the surface of the substance,
an optical medium (108) which is in direct contact with the first region (102) of the surface of the substance (101),
a detection device (106, 139) for detecting a response signal (SR),
wherein the apparatus (10) comprises a device (105) for emitting an electromagnetic measurement beam (112) which is arranged such that the emitted measurement beam (112) penetrates into the optical medium (108) and is totally reflected at the interface (GF) between the substance (101) and the optical medium (108), wherein the detection device (106) and the device (105) for emitting the electromagnetic measurement beam (112) are aligned with respect to one another such that the detection device (106) is configured to detect the reflected measurement beam which forms a time-dependent response signal (RS), wherein the amplitude of the response signal (RS) is influenced by the wavelength of the excitation beam and the intensity modulation thereof, and comprising a device (107, 147) for analyzing the substance on the basis of the detected response signal (SR),
the excitation transmission device (100) is mechanically fixedly connected directly - or indirectly by means of an adjustment apparatus (109) - to the optical medium (108), or the device for emitting a measurement beam (112) and/or the device (106) for detecting the reflected measurement beam and/or the excitation transmission device (100) is mechanically fixedly connected directly to the optical medium (108) and/or is coupled thereto by means of an optical waveguide (120).

2. The apparatus (10) according to claim 1,
**characterised in that**
during operation the measurement beam (112) and the excitation beam (SA) overlap on the interface (GF) of the optical medium (108) and the surface of the substance (101) at which the measurement beam (112) is reflected, and
the detection device (106) is a device for directly or indirectly detecting a deflection of the reflected measurement beam (112).

3. The apparatus (10) according to claim 1 or 2,
**characterised in that**
the detection device (106) for detecting a response signal (SR) detects a deformation and/or density change of the optical medium in a region adjacent to said first region as a result of the response signal,
the detection device has a piezo element connected to the optical medium or integrated therein as a detector for detecting a deformation or density change thereof, and/or
the detection device has temperature sensors as a detector for detecting the response signal, and/or
the apparatus has a device (104) for intensity modulation of the excitation light beam (SA) and
the detection device (106) is suitable for detecting a time-dependent response signal (SR) depending on the wavelength of the excitation light and/or the intensity modulation of the excitation light.

4. The apparatus (10) according to any one of the preceding claims,
**characterised in that**
the excitation transmission device (100) comprises two or more transmission elements (100a), in particular in the form of a one-, two- or multi-dimensional transmission element array, and
the two or more transmission elements (100a) each generate their own electromagnetic excitation beam and radiate it into the volume below said first region (102).

5. The apparatus (10) according to claim 4,
**characterised in that**
the wavelengths of the electromagnetic excitation beams of the two or more transmission elements (100a) differ, or
the excitation transmission device comprises two or more lasers, in particular in the form of one-, two- or multi-dimensional laser arrays, and/or two or more light-emitting diodes, in particular in the form of a one-, two- or multi-dimensional diode array.

6. The apparatus (10) according to claim 3,
**characterised in that**
the device (104) for intensity modulation comprises or is formed by an electrical modulation device which is electrically connected to the excitation transmission device (100) and electrically controls the latter, or
the device (104) for intensity modulation comprises at least one controlled mirror (133, 134) arranged in the beam path, or
the device (104) for intensity modulation comprises or is formed by at least one layer (138) arranged in the beam path and controllable with respect to its transparency.

7. The apparatus according to any one of the preceding claims,
**characterised in that**
the optical medium directly carries an imaging optical unit (128, 129) and/or an imaging optical unit (128, 129) is integrated into the optical medium.

8. The apparatus (10) according to any one of the preceding claims, in which the surface of the optical medium has a plurality of partial surfaces (110, 111) which are inclined with respect to one another and at which the measurement beam (112), in particular the measurement light beam, is reflected multiple times.

9. The apparatus (10) according to any one of the preceding claims,
**characterised in that**
one or more mirror surfaces (113, 114) for reflecting the measurement beam (112), in particular the measurement light beam, are provided in or on the optical medium (108), or
the excitation transmission device (100) and/or the device (105) for emitting the measurement beam and/or the detection device (106) are fastened directly to one another or to a common carrier (121).

10. The apparatus (10) according to claim 9,
**characterised in that**
the carrier (121) is formed by a printed circuit board, a metal plate or plastic plate or a housing (122) or housing part of the apparatus, or
the excitation transmission device comprises an integrated semiconductor component which has one or more laser elements and at least one micro-optical component and preferably additionally a modulation element.

11. The apparatus (10) according to claim 6,
**characterised in that**
the modulation element has at least one element, in particular a mirror, which is movable with respect to the remaining semiconductor component and controllable with respect to a position, or
the modulation element has a layer controllable in its radiation permeability.

12. The apparatus (10) according to claim 6,
**characterised in that**
the modulation device has an electronic control circuit for the modulation of the one or more laser elements.

13. A method for analyzing a substance (101) using an apparatus according to any one of claims 1 to 12, wherein in the method
one or more electromagnetic excitation beams (SA) having at least one excitation wavelength are generated by the excitation transmission device (100) by the at least partially simultaneous or successive operation of one or more laser emitters of a laser light source and are radiated into a volume (103) which is located in the substance below a first region (102) of the surface of the substance,
and wherein an electromagnetic measurement beam (112) which is totally reflected at the interface (GF) between the substance (101) and an optical medium (108) is emitted by the device (105) for emitting an electromagnetic measurement beam (112),
a time-dependent response signal (SR) is detected by the detection device (106), wherein the amplitude of the response signal is influenced by the wavelength of the excitation beam and its intensity modulation, and
the substance is analyzed by the device (107, 147) for analyzing the substance on the basis of the detected response signal (SR).

14. The method according to claim 13, **characterised in that** response signals in the form of temporal response signal patterns are determined successively using different modulation frequencies of the excitation transmission device (100) and that a plurality of response signal patterns at different modulation frequencies are linked to one another and that information specific to a depth range below the substance surface is obtained therefrom,
wherein response signal patterns at different modulation frequencies are each determined for different wavelengths of the excitation beam and in particular information specific to a depth range below the substance surface is obtained therefrom,
wherein preferably when a plurality of modulation frequencies of the pump beam are used at the same time, the detected signal is separated according to its frequencies by means of an analysis method, preferably a Fourier transform, and
in each case only the partial signal corresponding to the desired frequency is filtered out.

15. The method according to any one of the preceding claims 13-14,
**characterised in that**
a reflected measuring beam (112) forming the response signal (SR) is measured with the detection device (106),
or
in which the excitation beam is periodically amplitude-modulated with a first frequency and a frequency shift of the response signal relative to the first frequency is determined,
or
a dosing device for dispensing a substance into a patient's body is controlled depending on a substance concentration determined in the substance.

## Revendications

1. Dispositif (10) pour l'analyse d'une substance (101) avec
un dispositif émetteur d'excitation (100) sous la forme d'une source de lumière laser pour la production d'un ou plusieurs rayonnements d'excitation électromagnétiques (SA), avec au moins une longueur d'onde d'excitation, dans lequel le dispositif émetteur d'excitation (100) émet le ou les rayonnements d'excitation électromagnétiques (SA) dans un volume (103) qui se trouve dans la substance en dessous d'une première zone (102) de la surface de la substance,
un milieu optique (108) qui est en contact direct avec la première zone (102) de la surface de la substance (101),
un dispositif de détection (106, 139) pour la détection d'un signal de réaction (SR), dans lequel le dispositif (10) comprend un dispositif (105) pour l'émission d'un rayonnement de mesure électromagnétique (112), qui est disposé de sorte que le rayonnement de mesure (112) émis pénètre dans le milieu optique (108) et est réfléchi entièrement au niveau de la surface limite (GF) entre la substance (101) et le milieu optique (108), dans lequel le dispositif de détection (106) et le dispositif (105) pour l'émission du rayonnement de mesure électromagnétique (112) sont orientés entre eux de sorte que le dispositif de détection (106) est conçu pour la détection du rayonnement de mesure réfléchi, qui forme un signal de réaction (RS) dépendant du temps, dans lequel l'amplitude du signal de réaction (RS) est influencée par la longueur d'onde du rayonnement d'excitation et sa modulation d'intensité et
avec un dispositif (107, 147) pour l'analyse de la substance à l'aide du signal de réaction (SR) détecté, **caractérisé en ce que**
le dispositif émetteur d'excitation (100) est relié de manière fixe mécaniquement directement - ou indirectement par l'intermédiaire d'un dispositif d'ajustement (109) - avec le milieu optique (108) ou
le dispositif pour l'émission d'un rayonnement de mesure (112) et/ou le dispositif (106) pour la détection du rayonnement de mesure réfléchi et/ou le dispositif émetteur d'excitation (100) est relié de manière fixe mécaniquement directement avec le milieu optique et/ou est couplé à celui-ci au moyen d'une fibre optique (120).

2. Dispositif (100) selon la revendication 1,
**caractérisé en ce que**
pendant le fonctionnement, le rayonnement de mesure (112) et le rayonnement d'excitation (SA) se superposent sur la surface limite (GF) du milieu optique (108) et la surface de la substance (101), au niveau de laquelle le rayonnement de mesure (112) est réfléchi et
le dispositif de détection (106) est un dispositif pour la détection directe ou indirecte d'une déviation du rayonnement de mesure (112) réfléchi.

3. Dispositif (10) selon la revendication 1 ou 2,
**caractérisé en ce que**
le dispositif de détection (106), pour la détection d'un signal de réaction (SR) détecte une déformation et/ou une modification de la densité du milieu optique dans une zone voisine de la première zone mentionnée à la suite du signal de réaction ou
le dispositif de détection comprend un élément piézoélectrique relié avec le milieu optique ou intégré dans celui-ci, en tant que détecteur pour la détection d'une déformation d'une modification de la densité de celui-ci et/ou le dispositif de détection comprend des capteurs de température en tant que détecteur pour la détection du signal de réaction et/ou
le dispositif comprend un dispositif (104) pour la modulation d'intensité du rayonnement d'excitation (SA) et
le dispositif de détection (106) est conçu pour la détection d'un signal de réaction (SR) dépendant du temps en fonction de la longueur d'onde de la lumière d'excitation et/ou de la modulation d'intensité de la lumière d'excitation.

4. Dispositif (10) selon l'une des revendications précédentes,
**caractérisé en ce que**
le dispositif émetteur d'excitation (100) comprend deux éléments émetteurs (100a) ou plus, plus particulièrement sous la forme d'une matrice d'éléments émetteurs monodimensionnelles, bidimensionnelle ou multidimensionnelle et
les deux éléments émetteurs (100a) ou plus génèrent chacun leur propre rayonnement d'excitation électromagnétique et introduisent celui-ci dans le volume en dessous de la première zone (102) mentionnée.

5. Dispositif (10) selon la revendication 4,
**caractérisé en ce que**
les longueurs d'onde des rayonnements d'excitation électromagnétiques des deux éléments émetteurs (100a) ou plus sont différentes ou
le dispositif émetteur d'excitation comprend deux lasers ou plus, plus particulièrement sous la forme d'une matrice laser monodimensionnelle, bidimensionnelle ou multidimensionnelle et/ou deux diodes laser ou plus, plus particulièrement sous la forme d'une matrice de diodes monodimensionnelle, bidimensionnelle ou multidimensionnelle.

6. Dispositif (10) selon la revendication 3,
**caractérisé en ce que**
le dispositif (104) pour la modulation d'intensité comprend un dispositif de modulation électrique qui est relié électriquement avec le dispositif émetteur d'excitation (100) et contrôle celui-ci électriquement ou est constitué de celui-ci ou
le dispositif (104) pour la modulation d'intensité comprend au moins un miroir (133, 134) contrôlé disposé dans le trajet des rayons ou
le dispositif (104) pour la modulation d'intensité comprend au moins une couche (138) disposée dans le trajet des rayons, contrôlable par rapport à sa transparence ou est constitué de celle-ci.

7. Dispositif selon l'une des revendications précédentes,
**caractérisé en ce que**
le milieu optique supporte directement une optique de reproduction (128, 129) et/ou, dans le milieu optique, est intégré une optique de reproduction (128, 129).

8. Dispositif (10) selon l'une des revendications précédentes, dans lequel la surface du milieu optique comprend plusieurs surfaces partielles (110, 111) inclinées les unes par rapport aux autres, au niveau desquelles le rayonnement de mesure (112), plus particulièrement le rayon lumineux de mesure, est réfléchi plusieurs fois.

9. Dispositif (10) selon l'une des revendications précédentes,
**caractérisé en ce que**
dans ou sur le milieu optique (108), est prévu une ou plusieurs surfaces réfléchissantes (113, 114) pour la réflexion du rayonnement de mesure (112), plus particulièrement du rayon lumineux de mesure, ou
le dispositif émetteur d'excitation (100) et/ou le dispositif (105) pour l'émission du rayonnement de mesure et/ou le dispositif de détection (106) sont fixé directement les uns sur les autres ou sur un support commun (121).

10. Dispositif (10) selon la revendication 9,
**caractérisé en ce que**
le support (121) est constitué d'une carte de circuit imprimé, d'une plaque métallique ou d'une plaque en matière plastique ou d'un boîtier (122) ou d'une partie de boîtier du dispositif ou
le dispositif émetteur d'excitation comprend un composant semi-conducteur intégré qui comprend un ou plusieurs éléments laser ainsi qu'au moins un composant micro-optique et de préférence en outre un élément de modulation.

11. Dispositif (10) selon la revendication 6,
**caractérisé en ce que**
l'élément de modulation comprend au moins un élément mobile par rapport à l'autre composant semi-conducteur et contrôlable en ce qui concerne une position, plus particulièrement un miroir ou
l'élément de modulation comprend une couche contrôlable en ce qui concerne sa perméabilité au rayonnement.

12. Dispositif (10) selon la revendication 6,
**caractérisé en ce que**
le dispositif de modulation comprend un circuit de contrôle électronique pour la modulation du ou des éléments laser.

13. Procédé d'analyse d'une substance (101) à l'aide d'un dispositif selon l'une des revendications 1 à 12, dans lequel, dans le procédé
avec le dispositif émetteur d'excitation (100), un ou plusieurs rayonnements d'excitation électromagnétiques (SA) avec au moins une longueur d'onde d'excitation sont générés par le fonctionnement au moins partiellement simultané ou successif d'un ou plusieurs émetteurs lasers d'une source de lumière laser et sont introduits dans un volume (103), qui se trouve dans la substance en dessous d'une première zone (102) de la surface de la substance,
et dans lequel, avec le dispositif (105) pour l'émission d'un rayonnement de mesure électromagnétique (112), un rayon de mesure électromagnétique (112) est émis, qui est réfléchi entièrement au niveau de la surface limite (GF) entre la substance (101) et un milieu optique (108),
avec le dispositif de détection (106), un signal de réaction (SR) dépendant du temps est détecté, dans lequel l'amplitude du signal de réaction est influencé par la longueur d'onde du rayonnement d'excitation et sa modulation d'intensité et
avec le dispositif (105, 147) pour l'analyse de la substance, la substance est analysée à l'aide du signal de réaction (SR) détecté.

14. Procédé selon la revendication 13, **caractérisé en ce que**, en utilisant successivement différentes fréquences de modulation du dispositif émetteur d'excitation (100), sont déterminés des signaux de réaction sous la forme de tracés de signaux de réaction en fonction du temps et **en ce que** plusieurs tracés de signaux de réaction sont combinés entre eux en différentes fréquences de modulation et **en ce que** des informations spécifiques sont obtenues pour une zone en profondeur sous la surface de la substance, dans lequel les tracés de signaux de réaction à différentes fréquences de modulation sont déterminés chacun pour différentes longueurs d'ondes du rayonnement d'excitation et des informations spécifiques sont obtenues pour une zone en profondeur sous la surface de la substance,
dans lequel, de préférence, en utilisant plusieurs fréquences de modulation du rayonnement de pompage en même temps, le signal détecté est séparé en fonction de ses fréquences au moyen d'un procédé d'analyse, de préférence d'une transformation de Fourier et
seul le signal partiel qui correspond à la fréquence souhaitée est extrait par filtration.

15. Procédé selon l'une des revendications précédentes 13 - 14,
**caractérisé en ce que**
avec le dispositif de détection (106), un rayonnement de mesure (112) réfléchi formant le signal de réaction (SR) est mesuré
ou
dans lequel le rayonnement d'excitation est modulé en amplitude périodiquement avec une première fréquence et un décalage de fréquence du signal de réaction par rapport à la première fréquence est déterminé ou
en fonction d'une concentration de substance déterminée dans la substance, un dispositif de dosage est contrôlé pour la distribution d'une substance dans le corps d'un patient.
